# EUROPEAN PATENT APPLICATION

(11) **EP 4 700 125 A2**
(43) Date of publication of application: **25.02.2026**
(21) Application number: 24791740.4
(22) Date of filing: 27.02.2024
(51) Int. Cl.: C12N 15/113, A61K 31/713, A61K 48/00

(54) **OLIGOMERIC NUCLEIC ACID, AND PREPARATION METHOD THEREFORE AND USE THEREOF**

(30) Priority: 21.04.2023 CN 202310436173; 29.06.2023 CN 202310782933
(71) Applicant: Suzhou Siran Biotechnology Co., Ltd., Suzhou, Jiangsu 215000 (CN)
(72) Inventor: YANG, Zhiwei, Suzhou, Jiangsu 215000 (CN); LIU, Nan, Suzhou, Jiangsu 215000 (CN); HUANG, Minyin, Suzhou, Jiangsu 215000 (CN)
(74) Representative: Bryers Intellectual Property Ltd
(86) International application number: PCT/CN2024/078718
(87) International publication number: WO 2024/217152

(57) **Abstract**

The present disclosure relates to an oligonucleotide and a preparation method and use thereof. The present disclosure provides a single-stranded or double-stranded oligonucleotide, wherein each strand has 15 to 35 nucleotides, and at least one nucleotide position of the oligonucleotide other than the termini comprises a chemical modification represented by formula (II) or a tautomer modification thereof. The modification method of the present disclosure can minimize the impact on the on-target activity of siRNA while reducing its off-target effect.

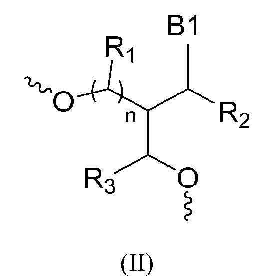

## Description

### FIELD OF TECHNOLOGY

The present disclosure belongs to the field of biomedical technology, and specifically relates to an oligonucleotide and a preparation method and use thereof.

### BACKGROUND

siRNA has very important prospects for drug development. However, in in vivo disclosures, siRNA often has varying degrees of off-target effects, where the siRNA targets non-target genes. An off-target effect similar to the microRNA pathway is one of the main causes of siRNA off-targeting, i.e., the inhibitory activity on mRNA produced by the complete or incomplete pairing between the seed region (positions 2-8 at the 5' end) of the siRNA antisense strand (AS strand) and the target mRNA. The off-target effect of one siRNA molecule may affect multiple mRNAs. Therefore, unpredictable toxic side effects may occur, which is the main reason for the toxic side effects of siRNA drugs.

To reduce off-target toxicity and improve the safety of siRNA drugs, specific sites in the seed region of the antisense strand of siRNA drugs can be chemically modified to reduce the off-target toxicity of small nucleic acid drugs. By introducing chemical modifications, the strength of the interaction between the seed region and the target gene can be adjusted, striving to reduce the off-target effect without affecting the silencing effect on the target mRNA (Mark K. Schlegel, Chirality Dependent Potency Enhancement and Structural Impact of Glycol Nucleic Acid Modification on siRNA. J. Am. Chem. Soc. 2017, 139, 8537-8546).

In the prior art, after introducing chemical modifications to the 5' end seed region of the siRNA antisense strand, for example, introducing GNA or UNA modifications to the 5' end seed region of the siRNA antisense strand, the off-target toxicity is reduced, but at the same time, a significant amount of on-target activity is also lost. Therefore, the prior art is in need of a chemical modification method that can minimize the impact on the on-target activity of siRNA while reducing its off-target effect.

### SUMMARY

An object of the present disclosure is to provide an oligonucleotide, and a preparation method and use thereof, which can minimize the impact on the on-target activity of siRNA while reducing its off-target effect.

To achieve the above object, the technical solution adopted by the present disclosure is:
A first aspect of the present disclosure provides a single-stranded or double-stranded oligonucleotide, wherein each strand has 15 to 35 nucleotides, and at least one nucleotide position of the oligonucleotide other than the termini comprises a chemical modification represented by formula (II) or a tautomer modification thereof, 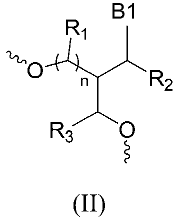
wherein, B1 is a natural nucleobase, a modified nucleobase, a universal base or an H atom;
*R*₁, *R*₂ and *R*₃ are each independently selected from H, OH, halogen, *NH*₂, *C*₁ - *C*₆ alkyl, *C*₁ - *C*₆ alkoxy, *C*₂ - *C*₆ alkenyl, *C*₂ - *C*₆ alkynyl, *S - CH*₃, *NCH*₃(*CH*₃), *OCH*₂*CH*₂*OCH*₃, and -O-alkylamino;
n is 1, 2 or 3;
wherein the chemical modification represented by formula (II) is not

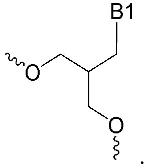

Preferably, B1 is a natural nucleobase, a modified nucleobase, a universal base or an H atom;
*R*₁, *R*₂ and *R*₃ are each independently selected from H, OH or *CH*₃;
n is 1 or 2.

According to some embodiments, the oligonucleotide is an siRNA, which comprises a sense strand and an antisense strand, each strand having 15 to 35 nucleotides, wherein the antisense strand comprises a chemical modification represented by formula (II-1), (II-2), (II-3) or (II-4) or a tautomer modification thereof at at least one nucleotide position from position 2 to position 8 of its 5' region, 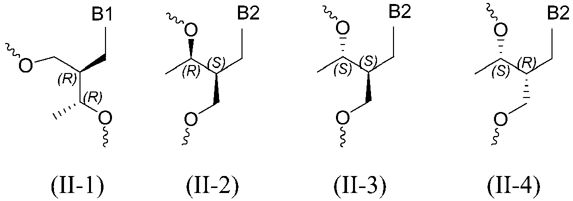 wherein: B1, B2 are each a natural nucleobase, a modified nucleobase, a universal base or an H atom.

Preferably, B1, B2 are each a modified or unmodified nucleobase.

According to some preferred embodiments, the chemical modification is selected from any of the following structures:

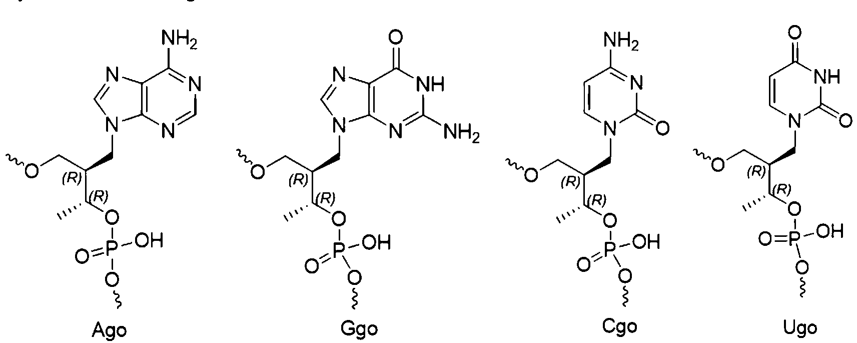

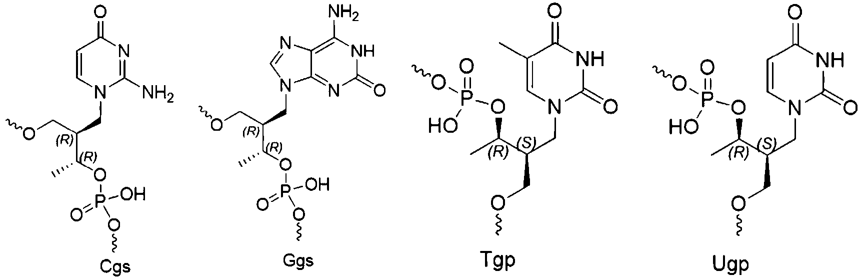

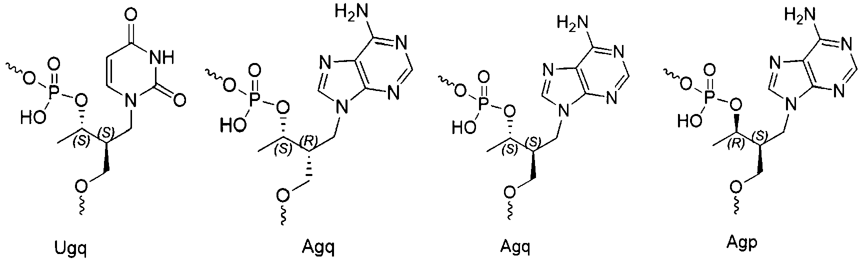

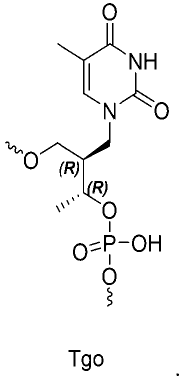

According to some preferred embodiments, the chemical modification is selected from any of the following structures: Ago, Ggo, Cgo, Ugo, Ggs, Tgo.

According to some embodiments, the strand comprises a chemical modification represented by formula (II), (II-1), (II-2), (II-3) or (II-4) or a tautomer modification thereof at at least one nucleotide position from position 5 to position 8 of its 5' region.

Further, the strand comprises a chemical modification represented by (II), (II-1), (II-2), (II-3) or (II-4) or a tautomer modification thereof at at least one nucleotide position of position 6 and 7 of its 5' region.

Further, the strand is the antisense strand of a double-stranded oligonucleotide, and comprises a chemical modification represented by Ago, Ggo, Cgo, Ugo, Ggs or Tgo or a tautomer modification thereof at at least one nucleotide position of position 6 and 7 of its 5' region.

Further, in addition to the nucleotide comprising the chemical modification represented by formula (II), (II-1), (II-2), (II-3) or (II-4) or a tautomer modification thereof, the strand further comprises at least one other modified nucleotide.

Still further, the other modified nucleotides are independently selected from: a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, and a 2'-substituted amino-modified nucleotide.

Preferably, the other modified nucleotides are independently selected from: a 2'-F modified nucleotide, a 2'-O-*CH*₃ modified nucleotide, a 2'-O-*CH*₂ *- CH*₂ *- O - CH*₃ modified nucleotide, a 2'-O-*CH*₂ *- CH = CH*₂ modified nucleotide, a 2'-*CH₂ - CH*₂ *- CH = CH*₂ modified nucleotide, and a 2'-deoxynucleotide;

More preferably, the other modified nucleotides are independently selected from: a 2'-F modified nucleotide and a 2'-O-*CH*₃ modified nucleotide.

Further, the oligonucleotide is an siRNA, which comprises a sense strand and an antisense strand, wherein the siRNA has at least one of the following characteristics:
(i) the antisense strand comprises 2, 3, 4, 5 or 6 2'-fluoro modifications;
(ii) the antisense strand comprises 1, 2, 3 or 4 phosphorothioate internucleotide linkages;
(iii)the sense strand comprises 2, 3, 4 or 5 2'-fluoro modifications;
(iv)the sense strand comprises 1, 2, 3 or 4 phosphorothioate internucleotide linkages.

Further, the siRNA comprises a duplex region of 12-40 nucleotide pairs in length.

According to some specific embodiments, the sense strand of the oligonucleotide is 5'-UmsGmsAmCmAfAmGfAfAfUmCmCmUmCmAmCmAmAmUm-3', and the antisense strand is 5'-AmsUfsUmGmUm(Ggo)AmGmGmAmUmUmCmUfUmGfUmCmAmsAmsCm-3'; or,
the sense strand of the oligonucleotide is 5'-UmsGmsAmCmAfAmGfAfAfUmCmCmUmCmAmCmAmAmUm-3', and the antisense strand is 5'-AmsUfsUmGmUmGf(Ago)GmGmAmUmUmCmUfUmGfUmCmAmsAmsCm-3'; or,
the sense strand of the oligonucleotide is 5'-CmsCmsUmUmGmAmGfGfCfAmUmAmCmUmUmCmAmAmAm -3', and the antisense strand is 5'-UmsUfsUmGmAm(Ago)GmUmAmUmGmCmCmUfCmAfAmGmGmsUmsUm -3'; or,
the sense strand of the oligonucleotide is 5'-CmsCmsUmUmGmAmGfGfCfAmUmAmCmUmUmCmAmAmAm-3', and the antisense strand is 5'-UmsUfsUmGmAmAf(Ggo)UmAmUmGmCmCmUfCmAfAmGmGmsUmsUm-3'; or,
the sense strand of the oligonucleotide is 5'-GmsUmsGmUmGmCmAfCfUf UmCmGmCmUmUmCmAmCmAm-3', and the antisense strand is 5'-UmsGfsUmGmAmAf(Ggo)CfGfAmAmGmUmGfCmAfCmAmCmsUmsUm-3'; or,
the sense strand of the oligonucleotide is 5'-AmsCmsUmGmAmAmGfCfAfUmUmUmGmAmUmGmCmAmAm-3', and the antisense strand is 5'-UmsUfsGmCmAm(Ugo)CmAmAmAmUmGmCmUfUmCfAmGmUmsGmsUm-3'; or,
the sense strand of the oligonucleotide is 5'-AmsCmsUmGmAmAmGfCfAfUmUmUmGmAmUmGmCmAmAm-3', and the antisense strand is 5'-UmsUfsGmCmAmUf(Cgo)AmAmAmUmGmCmUfUmCfAmGmUmsGmsUm-3'; or,
the sense strand of the oligonucleotide is 5'-AmsUmAmAmCmUmCfAfCfUmAmUmAmAmUmUmAmCmUm-3', and the antisense strand is 5'-AmsGfsUmAmAm(Ugo)UmAmUmAmGmUmGmAfGmUfUmAmUmsUmsUm-3'; or,
the sense strand of the oligonucleotide is 5'-AmsUmAmAmCmUmCfAfCfUmAmUmAmAmUmUmAmCmUm-3', and the antisense strand is 5'-AmsGfsUmAmAmUf(Ugo)AmUmAmGmUmGmAfGmUfUmAmUmsUmsUm-3'; or,
the sense strand of the oligonucleotide is 5'-GmsUmsGmUmGmCmAfCfUfUmCmGmCmUmUmCmAmCmsAms-3', and the antisense strand is 5'-VPUmsGfsUmGmAm(Ago)GmCmGmAmAmGmUmGfCmAfCmAmCmsUmsUm-3'; or,
the sense strand of the oligonucleotide is 5'-GmsUmsGmUmGmCmAfCfUfUmCmGmCmUmUmCmAmCmAm-3', and the antisense strand is 5'-UmsGfsUmGmAmAgoGmCmGmAmAmGmUmGfCmAfCmAmCmsUmsUm-3'.

According to other specific embodiments, the sense strand of the oligonucleotide is 5'-CmsCmsUmUmGmAmGfGfCfAmUmAmCmUmUmCmAmAmAm-3', and the antisense strand is 5'-UmsUfsUmGmAmAf(Ggs)UmAmUmGmCmCmUfCmAfAmGmGmsUmsUm -3'; or,
the sense strand of the oligonucleotide is 5'-GmsUmsGmUmGmCmAfCfUfUmCmGmCmUmUmCmAmCmAm -3', and the antisense strand is 5'-UmsGfsUmGmAmAf(Ggs)CfGfAmAmGmUmGfCmAfCmAmCmsUmsUm -3'.

A second aspect of the present disclosure provides an siRNA conjugate, which comprises the above single-stranded or double-stranded oligonucleotide and a conjugating group conjugated to the oligonucleotide.

Further, the conjugating group is linked to the 3' end and/or 5' end of the sense strand.

In some specific and preferred embodiments, the conjugating group is

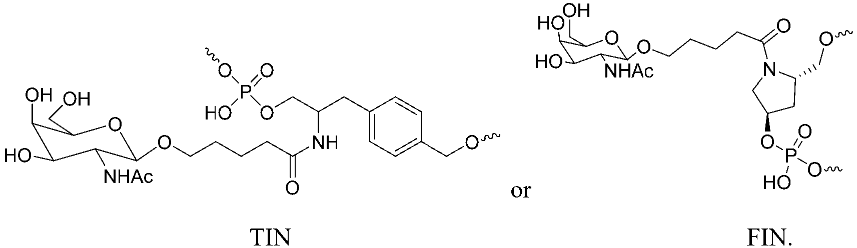

A third aspect of the present disclosure provides a pharmaceutical composition, which comprises the above single-stranded or double-stranded oligonucleotide or the above siRNA conjugate, and a pharmaceutically acceptable carrier.

A fourth aspect of the present disclosure provides a kit, which comprises the above single-stranded or double-stranded oligonucleotide, or the above siRNA conjugate, or the above pharmaceutical composition.

A fifth aspect of the present disclosure provides a compound represented by formula (IV) or a tautomer thereof, 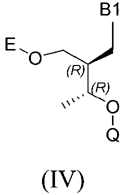
wherein, B1 is a natural nucleobase, a modified nucleobase, a universal base or an H atom;
E is a leaving group; preferably, E is MMTr or DMTr;
Q is a phosphorus-containing reactive group; preferably, Q is

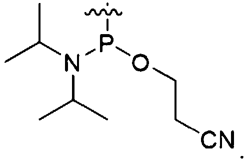

According to some specific and preferred embodiments, the compound represented by formula (IV) or a tautomer thereof is selected from any of the following structures:

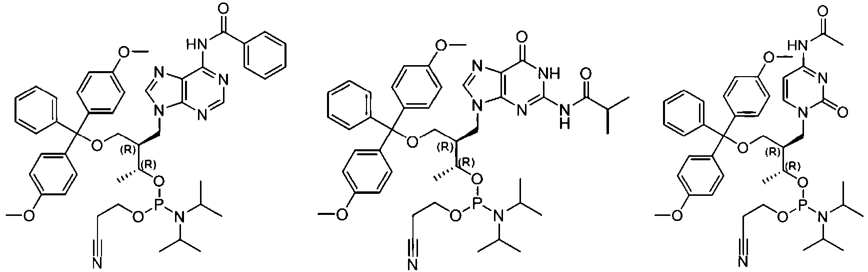

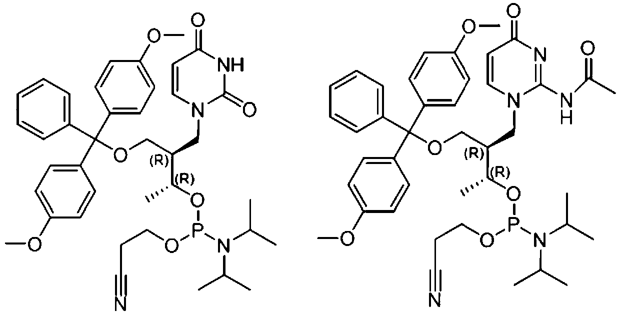

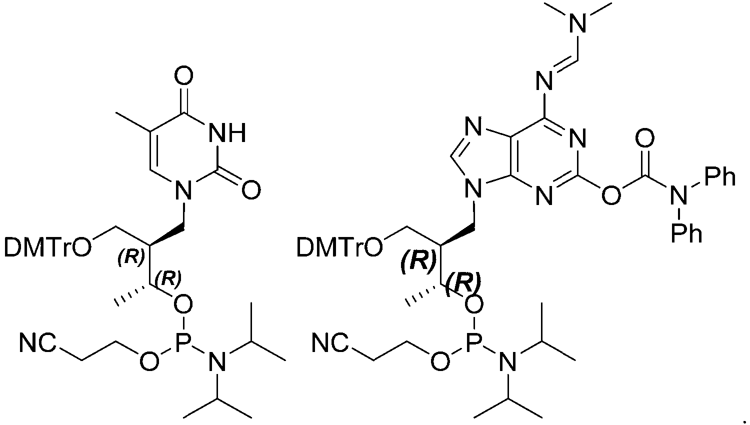

A sixth aspect of the present disclosure provides a method for preparing the above single-stranded or double-stranded oligonucleotide or the above siRNA conjugate, comprising the following steps:
1) synthesizing the compound represented by formula (IV) or a tautomer thereof as described above;
2) using the compound or tautomer thereof from step 1) to synthesize the oligonucleotide or siRNA conjugate.

A seventh aspect of the present disclosure provides a method for inhibiting a target gene in a cell, the method comprising the step of introducing the above single-stranded or double-stranded oligonucleotide or the above siRNA conjugate into the cell.

According to some embodiments, target genes include but are not limited to PD-L1, HBV, AGT, PCSK9, APOC3 or LPA, etc.

An eighth aspect of the present disclosure provides a method for modifying positions 2-8 of the antisense strand of an siRNA to reduce off-target activity, by introducing a chemical modification as represented by general formula (II), (II-1), (II-2), (II-3) or (II-4) or a tautomer modification thereof into positions 2-8 of the antisense strand of an siRNA molecule.

According to some specific embodiments, the chemical modification represented by general formula (II), (II-1), (II-2), (II-3) or (II-4) or a tautomer modification thereof is selected from any of the structures drawn above.

Further, the chemical modification represented by general formula (II), (II-1), (II-2), (II-3) or (II-4) or a tautomer modification thereof is selected from any of the structures Ago, Ggo, Cgo, Ugo, Ggs, Tgo.

Due to the disclosure of the above technical solutions, the present disclosure has the following advantages over the prior art:
The present disclosure, by introducing a special compound into the 5' end seed region of the siRNA antisense strand, minimizes the impact on the on-target activity of siRNA while reducing its off-target effect, thereby improving the safety of siRNA as a drug.

### BRIEF DESCRIPTION

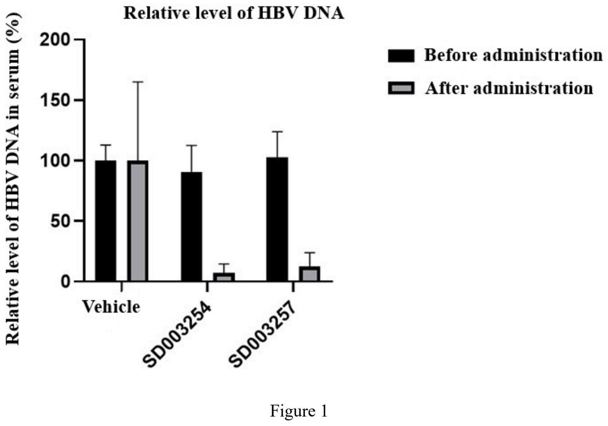

Figure 1 shows the level (%) of HBV DNA relative to the vehicle group.

### DETAILED DESCRIPTION

It should be noted that, unless otherwise defined, technical or scientific terms used in this disclosure shall have the ordinary meaning as understood by a person skilled in the art.

The experimental methods in the following examples, unless otherwise specified, are conventional methods. The raw materials, reagents, and materials used in the following examples, unless otherwise specified, are commercially available products.

When used herein and in the appended claims, the singular forms "a", "an", "another", and "the" include plural referents unless the context clearly dictates otherwise.

### Definitions

In the context of the present disclosure, the uppercase letters A, U, C, G represent the base composition of a nucleotide; the lowercase letter m indicates that the nucleotide immediately to the left of the letter m is a 2'-methoxy-modified nucleotide; the lowercase letter f indicates that the nucleotide immediately to the left of the letter f is a 2'-fluoro-modified nucleotide; the lowercase letter s indicates that the two nucleotides immediately to the left and right of the letter s are linked by a phosphorothioate group; the letter combination VP indicates that the nucleotide immediately to the right of the letter combination VP is a vinylphosphonate-modified nucleotide, as shown below.

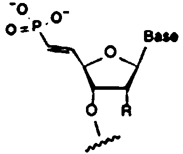

As used herein, the term "natural nucleobase" refers to a nucleobase that has not been modified from its naturally occurring form in RNA or DNA. Examples of "natural nucleobases" include the purine nucleobases adenine (A) and guanine (G), and the pyrimidine nucleobases thymine (T), cytosine (C), and uracil (U). In addition to "natural nucleobases", many modified nucleobases or nucleobase analogs known to those skilled in the art are suitable for the compounds described herein.

As used herein, the term "modified nucleobase" refers to a nucleobase that is structurally very similar to a parent nucleobase, such as 7-deazapurine, 5-methylcytosine, or a G-clamp.

As used herein, a universal nucleic acid base is a base that can form a complementary pair with at least two common bases, for example, hypoxanthine (whose nucleoside is inosine), which can pair with any of A, T, G, or C, with binding affinities of I:C > I:A > I:G > I:T; or for example, 5-bromouridine (BrU), which can pair with A or G; optionally, other universal bases capable of forming complementary pairs with at least two common bases may also be used in the present disclosure, such as 3-nitropyrrole, 5-nitroindole, 7-azaindole, and the like.

As used herein, the term "seed region" is the region of the antisense strand of an RNAi agent that is responsible for recognizing the target mRNA, and corresponds to, for example, nucleotides 2-8 from the 5' end of the antisense strand.

As used herein, the term "nucleotide position" refers to the position of a nucleotide in an oligonucleotide as counted from the 5' end. For example, nucleotide position 1 refers to the 5'-terminal nucleotide of the oligonucleotide.

As used herein, "oligonucleotide" refers to a polymeric form of nucleotides ranging from 2 to 2500 nucleotides. An oligonucleotide can be single-stranded or double-stranded. In certain embodiments, the oligonucleotide has 500 to 1500 nucleotides, typically, for example, where the oligonucleotide is used for gene therapy. In certain embodiments, the oligonucleotide is single-stranded or double-stranded and has 7 to 100 nucleotides. In certain embodiments, the oligonucleotide is single-stranded or double-stranded and has 15 to 100 nucleotides. In another embodiment, the oligonucleotide is single-stranded or double-stranded and has 15 to 50 nucleotides, typically, for example, where the oligonucleotide is a nucleic acid inhibitor molecule. In another embodiment, the oligonucleotide is single-stranded or double-stranded and has 25 to 40 nucleotides, typically, for example, where the oligonucleotide is a nucleic acid inhibitor molecule. In yet another embodiment, the oligonucleotide is single-stranded or double-stranded and has 19 to 40 or 19 to 25 nucleotides, typically, for example, where the oligonucleotide is a double-stranded nucleic acid inhibitor molecule and forms a double helix with at least 18 to 25 base pairs. In other embodiments, the oligonucleotide is single-stranded and has 15 to 25 nucleotides, typically, for example, where the oligonucleotide is a single-stranded RNAi inhibitor molecule. Typically, as described herein, the oligonucleotide contains one or more phosphorus-containing internucleotide linking groups. In other embodiments, as described herein, the internucleotide linking group is a phosphoramidite group.

As used herein, a "2'-fluoro-modified nucleotide" refers to a nucleotide in which the hydroxyl group at the 2' position of the ribose is replaced by fluorine, having the structure shown in formula (7) below.

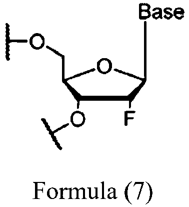

Herein, base represents a base such as A, U, G, C or T.

Similarly, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, and a 2'-substituted amino-modified nucleotide refer to nucleotides formed by the substitution of the hydroxyl group at the 2' position of the ribose with the corresponding group.

As used herein, "conjugation" refers to the connection of two or more chemical moieties, each having a specific function, to each other in a covalent manner; accordingly, a "conjugate" refers to a compound formed by the covalent connection of these individual chemical moieties. Further, an "siRNA conjugate" represents a compound formed by covalently linking one or more chemical moieties with specific functions to an siRNA.

In the context of the present disclosure, particularly in describing the preparation methods for the siRNA, siRNA-containing compositions, or siRNA conjugates of this disclosure, unless otherwise specified, the term nucleoside monomer refers to the unmodified or modified RNA phosphoramidites (sometimes RNA phosphoramidites are also called nucleoside phosphoramidites) used in phosphoramidite solid-phase synthesis, according to the type and sequence of nucleotides in the siRNA or siRNA conjugate to be prepared. Phosphoramidite solid-phase synthesis is a method well-known to those skilled in the art for RNA synthesis. The nucleoside monomers used in this disclosure are all commercially available.

In the present disclosure, a "non-terminal position of the oligonucleotide" refers to any position other than the 5'- and 3'-termini of the oligonucleotide, in other words, any position other than the first nucleotide and the last nucleotide (in the 5'-3' direction) of the oligonucleotide.

The siRNA of the present disclosure contains nucleotide groups as the basic structural unit. It is well known to those skilled in the art that the nucleotide group contains a phosphate group, a ribose group, and a base.

The present disclosure introduces one of the compounds of this disclosure for chemical modification in the 5' end seed region of the siRNA antisense strand, which reduces the off-target effect of the siRNA while minimizing the impact on its on-target activity, thereby improving the safety of siRNA as a drug.

In the prior art, GNA or UNA modifications are typically introduced into the 5' end seed region of the siRNA antisense strand, which reduces off-target toxicity but also results in a significant loss of on-target activity.

Wherein, UNA is as shown in formula (3), and GNA is as shown in formula (4).

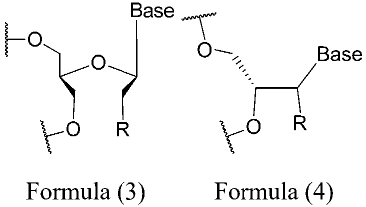

In the above formulas (3) and (4), R is selected from H, OH or alkoxy (O-alkyl).

In the examples of the present disclosure (including in vitro and in vivo experiments), different siRNA sequences targeting multiple targets were designed, and the on-target and off-target activities of siRNAs were compared when the chemical modification of the present disclosure and a GNA modification were separately introduced at the same position in the 5' end seed region of siRNA antisense strands composed of the same base sequence. The results show that introducing both the chemical modification of the present disclosure and the GNA modification can reduce the off-target effect of siRNA, improving its safety as a drug; however, siRNA with the chemical modification of the present disclosure has better on-target activity compared to the GNA-modified one, which indicates that introducing the chemical modification of the present disclosure can minimize the impact on the on-target activity of siRNA.

The present disclosure provides many chemical modifications, such as Ago, Ggo, Cgo, Ugo, Cgs, Ggs, etc. Introducing one of these chemical modifications at at least one nucleotide position from position 2 to position 8 of the 5' region of the antisense strand can retain on-target activity substantially the same as the original sequence and significantly reduce off-target activity.

In one embodiment, the antisense strand comprises a chemical modification represented by formula (II), (II-1), (II-2), (II-3) or (II-4) as defined above or a tautomer modification thereof at at least one nucleotide position from position 5 to position 8 of its 5' region;

Preferably, the antisense strand comprises a chemical modification represented by formula (II), (II-1), (II-2), (II-3), (II-4) as defined above or a tautomer modification thereof at at least one nucleotide position of position 6 and 7 of its 5' region.

In the examples of the present disclosure (including in vitro and in vivo experiments), it was verified that having the above chemical modification at the nucleotide position of the 6th or 7th position of the 5' end seed region of the siRNA antisense strand can both reduce the off-target effect of the siRNA and minimize the impact on its on-target activity.

In a preferred embodiment of the present disclosure, in addition to the nucleotide comprising the chemical modification represented by formula (II), (II-1), (II-2), (II-3), (II-4) as defined above or a tautomer modification thereof, at least one of the sense strand and/or the antisense strand also comprises at least one other modified nucleotide;

Preferably, in addition to the nucleotide comprising the chemical modification represented by formula (II), (II-1), (II-2), (II-3), (II-4) as defined above or a tautomer modification thereof, the remaining nucleotides of the sense strand and/or the antisense strand are other modified nucleotides.

In some embodiments, the other modified nucleotides are independently selected from: a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, and a 2'-substituted amino-modified nucleotide.

Preferably, the other modified nucleotides are independently selected from: a 2'-F modified nucleotide, a 2'-O-*CH*₃ modified nucleotide, a 2'-O-*CH*₂ *- CH*₂ *- O - CH*₃ modified nucleotide, a 2'-O-*CH*₂ *- CH = CH*₂ modified nucleotide, a 2'*-CH₂ - CH*₂ *- CH = CH*₂ modified nucleotide, and a 2'-deoxynucleotide;

More preferably, the other modified nucleotides are independently selected from: a 2'-F modified nucleotide and a 2'-O-*CH*₃ modified nucleotide.

In some embodiments, the 2'-alkyl-modified nucleotide is a methoxy-modified nucleotide (2'-OMe), as shown in formula (8).

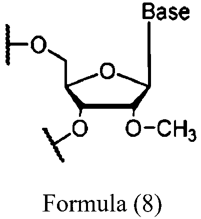

Wherein, Base represents a base, such as A, U, G, C or T.

In some embodiments, the single-stranded or double-stranded oligonucleotide/siRNA has at least one of the following characteristics:
(i) the antisense strand comprises 2, 3, 4, 5 or 6 2'-fluoro modifications;
(ii) the antisense strand comprises 1, 2, 3 or 4 phosphorothioate internucleotide linkages;
(iii)the sense strand comprises 2, 3, 4 or 5 2'-fluoro modifications;
(iv)the sense strand comprises 1, 2, 3 or 4 phosphorothioate internucleotide linkages, with the structure as shown in formula (1): 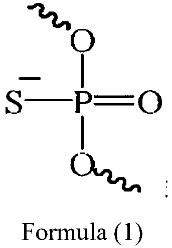
(vi)the siRNA comprises at least four 2'-fluoro modifications;
(vii) the siRNA comprises a duplex region of 12-40 nucleotide pairs in length.

### 2. siRNA Conjugate of the Present Disclosure

The present disclosure provides an siRNA conjugate, which comprises the above single-stranded or double-stranded oligonucleotide/siRNA and a conjugating group conjugated to the siRNA.

Preferably, the conjugating group comprises a pharmaceutically acceptable targeting group and a linker, and the single-stranded or double-stranded oligonucleotide/siRNA, the linker, and the targeting group are sequentially linked covalently or non-covalently.

In a preferred embodiment of the present disclosure, the conjugating group conjugated to the siRNA is TIN or FIN, wherein the synthesis of FIN can be found in US2022/0008541A1.

### 3. Pharmaceutical Composition and Kit of the Present Disclosure

The present disclosure provides a pharmaceutical composition, said pharmaceutical composition containing the single-stranded or double-stranded oligonucleotide/siRNA or siRNA conjugate as described above as an active ingredient and a pharmaceutically acceptable carrier.

The pharmaceutically acceptable carrier can be a carrier conventionally used in the field of siRNA administration, such as but not limited to magnetic nanoparticles (e.g., nanoparticles based on *Fe*₃*O*₄ or *Fe*₂*O*₃), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), and poly(2-dimethylaminoethyl methacrylate) (PDMAEMA), and derivatives thereof, in one or more forms.

In the pharmaceutical composition, there are no special requirements for the content of the single-stranded or double-stranded oligonucleotide/siRNA or siRNA conjugate and the pharmaceutically acceptable carrier, which can be the conventional content of each component.

The present disclosure provides a kit, which comprises the above single-stranded or double-stranded oligonucleotide/siRNA or the above siRNA conjugate or the above pharmaceutical composition.

In some embodiments, the kit described herein may provide the single-stranded or double-stranded oligonucleotide/siRNA or siRNA conjugate in one container. In some embodiments, the kit described herein may comprise a container providing a pharmaceutically acceptable excipient. In some embodiments, the kit may also contain other ingredients, such as stabilizers or preservatives. In some embodiments, the kit described herein may comprise at least one other therapeutic agent in a container other than the one providing the single-stranded or double-stranded oligonucleotide/siRNA or siRNA conjugate described herein. In some embodiments, the kit may comprise instructions for mixing the single-stranded or double-stranded oligonucleotide/siRNA or siRNA conjugate with a pharmaceutically acceptable carrier and/or excipient or other ingredients (if any).

In the kit of the present disclosure, the single-stranded or double-stranded oligonucleotide/siRNA, siRNA conjugate and pharmaceutically acceptable carrier and/or excipient, and/or pharmaceutical composition, and/or pharmaceutically acceptable carrier and/or excipient can be provided in any form, such as liquid form, dry form, or lyophilized form. In some embodiments, the single-stranded or double-stranded oligonucleotide/siRNA, siRNA conjugate and pharmaceutically acceptable carrier and/or excipient, and the pharmaceutical composition and pharmaceutically acceptable carrier and/or excipient are substantially pure and/or sterile. In some embodiments, sterile water may be provided in the kit of the present disclosure.

The technical solutions provided by the present disclosure will be further described below in conjunction with specific examples. The following examples are only for illustrating the present disclosure and do not limit the protection scope of the present disclosure.

### Example 1: Preparation of Compound SA000001

### 1.1 Preparation of Intermediate 2

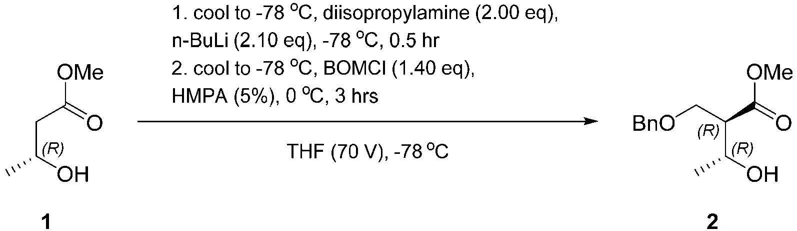

Anhydrous diisopropylamine (16.2 g, 160.0 mmol, 22.6 mL, 2.0 equiv) was dissolved in 350 mL of anhydrous tetrahydrofuran, cooled to between -70°C and -78°C, and protected by nitrogen displacement. n-Butyllithium solution (2.5 M, 67.1 mL) was added dropwise (slowly, over at least 10 minutes), and the reaction mixture was stirred for another half hour between -70°C and -78°C. Compound 1 (9.44 g, 79.9 mmol, 9.17 mL, 1.0 equiv) was dissolved in 175 mL of anhydrous tetrahydrofuran, cooled to between -70°C and - 78°C, and the previously prepared solution was added dropwise under nitrogen displacement protection (slowly, over at least 10 minutes). This reaction mixture was stirred for another half hour between -70°C and -78°C. While maintaining the temperature, hexamethylphosphoramide (26.1 g, 146 mmol, 25.6 mL, 1.82 equiv) and benzyl chloromethyl ether (17.5 g, 112 mmol, 15.5 mL, 1.4 equiv) were slowly added dropwise to the reaction mixture (slowly, over at least 10 minutes). After the addition was complete, the mixture was warmed to 0°C and stirred for 3 hours. TLC and LCMS indicated the disappearance of compound 1. The reaction was quenched by adding 600 mL of saturated ammonium chloride solution in two batches. The mixture was extracted with 200 mL of methyl tert-butyl ether. The organic phase was collected, washed with saturated brine, dried over anhydrous sodium sulfate, filtered, and the filtrate was concentrated. The resulting crude product was purified by silica gel column chromatography (petroleum ether/ethyl acetate = 100/1 to 1/1) to obtain pale brown compound 2 (9.69 g, 40.7 mmol, 51% yield).

¹H NMR (400 MHz, *CDCl*₃): *δ* 7.46-7.28 (m, 10H), 4.58-4.46 (m, 2H), 4.19-4.08 (m, 1H), 3.82-3.67 (m, 5H), 2.77 (q, *J =* 6.1 Hz, 1H), 1.24 (d, *J =* 6.5 Hz, 4H).

LC-MS: *C*₁₃*H*₁₈*O*₄, MW 238.1, 239.1 (M+H).

### 1.2 Preparation of Intermediate 3

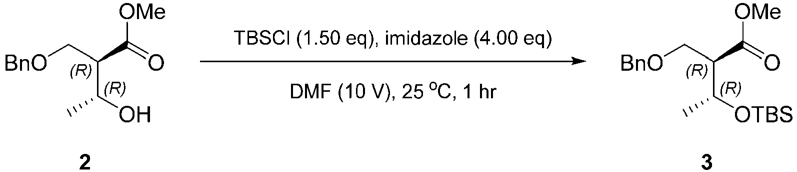

Compound 2 (14.7 g, 61.7 mmol, 1.0 equiv) was dissolved in 150 mL of dichloromethane. Under nitrogen protection at room temperature, imidazole (16.8 g, 247.0 mmol, 4.0 equiv) and tert-butyldimethylsilyl chloride (27.9 g, 185.0 mmol, 22.7 mL, 3.0 equiv) were added. The reaction mixture was stirred at room temperature for one hour. TLC and LCMS indicated the disappearance of compound 2. 100 mL of dichloromethane was added to the reaction mixture, which was then washed twice with 400 mL of saturated brine. The organic phase was dried, filtered, and concentrated. The resulting crude product was purified by silica gel column chromatography (petroleum ether/ethyl acetate = 100/1 to 10/1) to obtain pale yellow oily compound 3 (12.0 g, 34.0 mmol, 55% yield).

¹H NMR (400 MHz, *CDCl*₃): *δ* 7.40-7.25 (m, 5H), 4.52 (d, *J* = 2.4 Hz, 2H), 4.10 (t, *J =* 6.3 Hz, 1H), 3.75-3.65 (m, 4H), 3.59 (dd, *J* = 9.2, 5.3 Hz, 1H), 2.79 (dt, *J =* 8.4, 5.9 Hz, 1H), 1.17 (d, *J* = 6.2 Hz, 3H), 0.86 (s, 9H), 0.04 (d, *J* = 8.6 Hz, 6H).

LC-MS: *C*₁₉*H*₃₂*O*₄*Si*, MW 352.1, 353.2 (M+H).

### 1.3 Preparation of Intermediate 4

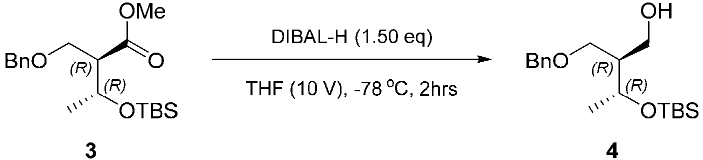

Compound 3 (11.1 g, 31.5 mmol, 1.0 equiv) was dissolved in tetrahydrofuran, cooled to between -70°C and -60°C. Under nitrogen protection, diisobutylaluminium hydride (1.0 M, 69.3 mL, 2.2 equiv) was added dropwise. The mixture was stirred at this temperature for two hours. LCMS indicated the disappearance of compound 3. The temperature was raised to 0°C, 20 mL of ethyl acetate was added, and the reaction was quenched with 100 mL of potassium sodium tartrate solution, followed by stirring for another half hour. The mixture was washed with 100 mL of saturated brine, dried over anhydrous sodium sulfate, filtered, and concentrated to obtain pale yellow oily compound 4 (9.75 g, 30.0 mmol, 95% yield).

¹H NMR (400 MHz, *CDCl*₃): *δ* 7.40-7.27 (m, 5H), 4.59-4.45 (m, 2H), 4.19 (dd, *J* = 6.2, 3.7 Hz, 1H), 4.01 (dd, *J =* 11.3, 4.0 Hz, 1H), 3.78-3.60 (m, 3H), 1.78-1.68 (m, 1H), 1.29-1.21 (m, 3H), 0.95-0.87 (m, 9H), 0.12-0.02 (m, 6H).

LC-MS: *C*₁₈*H*₃₂*O*₃*Si*, MW 324.1, 325.2 (M+H).

### 1.4 Preparation of Intermediate 5

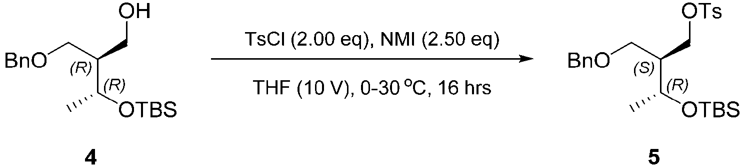

Compound 4 (9.75 g, 30.0 mmol, 1.0 equiv) was dissolved in tetrahydrofuran, cooled to 0°C. Under nitrogen protection, p-toluenesulfonyl chloride (11.5 g, 60.1 mmol, 2.0 equiv) and methylimidazole (6.17 g, 75.1 mmol, 5.99 mL, 2.5 equiv) were added dropwise. After the addition, the mixture was warmed to room temperature and stirred for 16 hours. LCMS indicated the disappearance of compound 4. 20 mL of ethyl acetate was added to the reaction mixture, and the reaction was quenched with 100 mL of potassium sodium tartrate solution in an ice bath, followed by stirring for another half hour. The mixture was washed with 100 mL of saturated brine, dried over anhydrous sodium sulfate, filtered, and concentrated to obtain the crude product, pale yellow oily compound 5 (13.7 g, 28.6 mmol, 95% yield).

¹H NMR (400 MHz, *CDCl*₃): *δ* 7.89-7.68 (m, 2H), 7.42-7.17 (m, 10H), 4.47-4.33 (m, 2H), 4.23 (dd, *J =* 9.6, 5.1 Hz, 1H), 4.13-4.06 (m, 1H), 4.01-3.94 (m, 1H), 3.52-3.34 (m, 2H), 2.43 (s, 3H), 2.02-1.89 (m, 1H), 1.10 (d, *J =* 6.3 Hz, 3H), 0.81 (s, 8H), 0.04-0.05 (m, 6H).

LC-MS: *C*₂₅*H*₃₈*O*₅*SSi*, MW 478.1, 479.2 (M+H).

### 1.5 Preparation of Intermediate 6

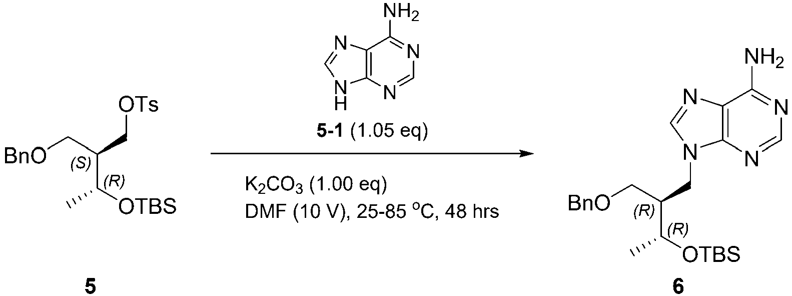

Under nitrogen protection, compound 5 (13.7 g, 28.6 mmol, 1.0 equiv) and 35 mL of acetonitrile were added to a dry reaction flask. The acetonitrile was distilled off at 35-40°C to remove water from compound 5. In another clean, dry reaction flask under nitrogen protection, 80 mL of N,N-dimethylformamide, compound 5-1 (9H-purin-6-amine) (4.25 g, 31.5 mmol, 1.1 equiv), and potassium carbonate (3.96 g, 28.6 mmol, 1.0 equiv) were added. The mixture was heated to 95-100°C and stirred for half an hour. At this temperature, a solution of compound 5 (13.7 g, 28.6 mmol, 1.0 equiv) in N,N-dimethylformamide (60 mL) was added dropwise to the reaction mixture, and stirring was continued for 12 hours. LCMS indicated the disappearance of compound 5. The reaction mixture was cooled to room temperature, 200 mL of ethyl acetate was added, and the resulting solution was washed sequentially with 200 mL of sodium bicarbonate solution and 100 mL of saturated brine. The organic phase was dried over anhydrous sodium sulfate, filtered, and the filtrate was concentrated. The resulting crude product was purified by silica gel column chromatography (petroleum ether/ethyl acetate = 20/1 to 10/1) to obtain pale yellow oily compound 6 (6.2 g, 14.0 mmol, 49% yield).

¹H NMR (400 MHz, *CDCl*₃): *δ* 8.37 (s, 1H), 7.75 (s, 1H), 7.39-7.24 (m, 6H), 5.66 (br, s, 2H), 4.47-4.34 (m, 3H), 4.21 (dd, *J* = 14.1, 8.8 Hz, 1H), 4.07 (dd, *J* = 6.2, 4.9 Hz, 1H), 3.42-3.34 (m, 2H), 2.10-1.96 (m, 1H), 1.19 (d, *J* = 6.4 Hz, 3H), 0.90 (s, 9H), 0.06 (d, *J* = 6.0 Hz, 6H).

LC-MS: *C*₂₃*H*₃₅*N*₅*O*₂*Si*, MW 441.1, 442.3 (M+H).

### 1.6 Preparation of Intermediate 7

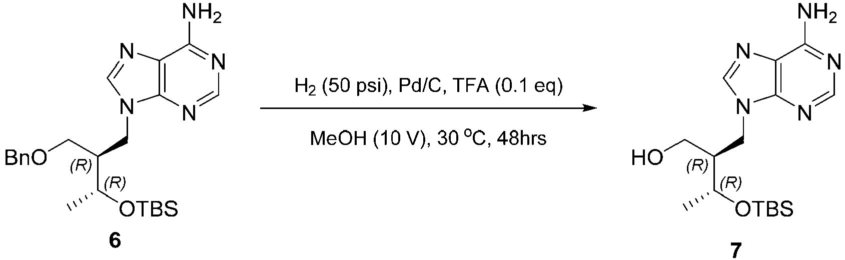

At room temperature, palladium on carbon (3.0 g, 10% purity) was dissolved in 25 mL of methanol. A solution of compound 6 (5.2 g, 11.8 mmol, 1.0 equiv) in 25 mL of methanol and trifluoroacetic acid (134.0 mg, 1.18 mmol, 87.2 uL) were added. The reaction mixture was stirred under 50 psi hydrogen pressure for 48 hours. LCMS indicated the disappearance of compound 6. The reaction mixture was filtered and concentrated to obtain the crude product, pale yellow oily compound 7 (3.9 g, 11.1 mmol).

¹H NMR (400 MHz, *CDCl*₃): *δ* 8.21 (s, 1H), 8.12 (s, 1H), 4.50-4.36 (m, 1H), 4.33-4.22 (m, 1H), 4.15-4.02 (m, 1H), 3.53 (d, *J =* 5.9 Hz, 2H), 2.21-2.09 (m, 1H), 1.25 (d, *J =* 6.4 Hz, 3H), 0.88 (s, 9H), 0.05 (d, *J* = 3.1 Hz, 6H).

LC-MS: *C*₁₆*H*₂₉*N*₅*O*₂*Si*, MW 351.1, 352.2 (M+1).

### 1.7 Preparation of Intermediate 8

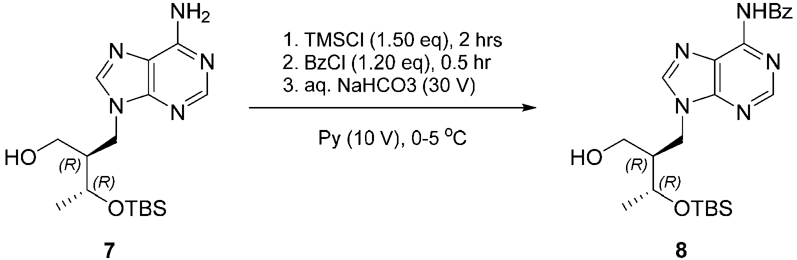

Compound 7 (3.9 g, 11.1 mmol, 1.0 equiv) was placed in a clean, dry reaction flask. Under nitrogen protection, 10 mL of pyridine was added, and the pyridine was distilled off by heating. This operation was repeated once to remove water from compound 7.28 mL of pyridine was added, and trimethylsilyl chloride (4.8 g, 44.4 mmol, 5.63 mL, 4.0 equiv) was added in an ice bath. The mixture was warmed to room temperature and stirred for two hours. TLC indicated the disappearance of compound 7. Benzoyl chloride (6.2 g, 44.4 mmol, 5.15 mL, 4.0 equiv) was added in an ice bath, and stirring was continued for 2 hours. TLC indicated the disappearance of the starting material. 60 mL of water and 90 mL of concentrated ammonia water were added dropwise, and stirring was continued for half an hour. The reaction mixture was extracted with 250 mL of ethyl acetate. The organic phase was washed with saturated brine, dried, filtered, and concentrated. The resulting crude product was purified by silica gel column chromatography (eluent system: dichloromethane/methanol = 50/1 to 10/1) to obtain pale yellow oily compound 8 (4.0 g, 8.78 mmol, 79% yield).

¹H NMR (400 MHz, *CDCl*₃): *δ* 9.22-8.90 (m, 1H), 8.79 (s, 1H), 8.07-8.04 (m, 2H), 8.03 (d, *J =* 1.4 Hz, 1H), 7.65-7.59 (m, 1H), 7.57-7.50 (m, 2H), 4.62-4.50 (m, 1H), 4.42 (dd, *J* = 14.3, 8.6 Hz, 1H), 4.24-4.09 (m, 1H), 4.05 (d, *J =* 6.3 Hz, 1H), 3.59-3.48 (m, 1H), 3.47-3.35 (m, 1H), 2.04-1.98 (m, 1H), 1.31 (d, *J =* 6.3 Hz, 3H), 0.98-0.84 (m, 9H), 0.11 (d, *J =* 10.1 Hz, 5H).

LC-MS: *C*₂₃*H*₃₃*N*₅*O*₃*Si*, MW 455.2, 456.3 (M+H).

### 1.8 Preparation of Intermediate 9

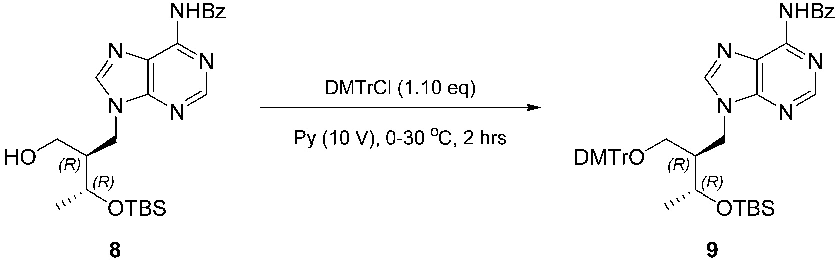

Compound 8 (4.0 g, 8.78 mmol, 1.0 equiv) was placed in a clean, dry reaction flask. Under nitrogen protection, 10 mL of pyridine was added, and the pyridine was distilled off by heating. This operation was repeated once to remove water from compound 8. 28 mL of pyridine was added, and 4,4'-dimethoxytrityl chloride (3.27 g, 9.66 mmol, 1.1 equiv) was added in an ice bath. The mixture was warmed to room temperature and stirred for 1 hour. TLC indicated the disappearance of compound 8. 150 mL of ethyl acetate was added to the reaction mixture, which was then washed with 200 mL of sodium bicarbonate solution and 150 mL of saturated brine. The organic phase was dried, filtered, and the filtrate was concentrated. The resulting crude product was purified by silica gel column chromatography (gradient elution: petroleum ether/ethyl acetate = 10/1 to 0/1) to obtain pale yellow oily compound 9 (5.91 g, 7.8 mmol, 88% yield).

¹H NMR (400 MHz, *CDCl*₃): *δ* 9.07 (br s, 1H), 8.82 (s, 1H), 8.04 (d, *J* = 7.1 Hz, 2H), 7.80 (s, 1H), 7.65-7.58 (m, 1H), 7.57-7.50 (m, 2H), 7.28-7.13 (m, 9H), 6.81-6.69 (m, 4H), 4.52-4.43 (m, 1H), 4.25 (dd, *J =* 14.2, 8.6 Hz, 1H), 4.07 (dd, *J =* 6.3, 4.2 Hz, 1H), 3.76 (d, *J* = 4.5 Hz, 6H), 3.22 (dd, *J =* 9.8, 5.4 Hz, 1H), 3.04 (dd, *J =* 9.8, 6.1 Hz, 1H), 2.34 (dd, *J* = 8.1, 4.5 Hz, 1H), 1.19 (d, *J =* 6.3 Hz, 3H), 0.90-0.77 (m, 9H), 0.07-0.09 (m, 6H).

LC-MS: *C*₄₄*H*₅₁*N*₅*O*₅*Si*, MW 757.3, 758.4 (M+H).

### 1.9 Preparation of Intermediate 10

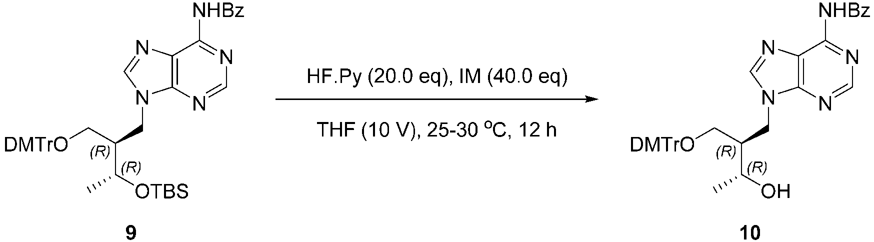

Compound 9 (3.00 g, 3.96 mmol, 1.0 equiv) was dissolved in tetrahydrofuran. Under nitrogen protection and in an ice bath, hydrogen fluoride-pyridine salt (2.26 g, 79.2 mmol, 2.1 mL, 20.0 equiv) and imidazole (10.8 g, 158.0 mmol, 40.0 equiv) were added. The mixture was warmed to room temperature and stirred for 2 hours. LCMS indicated the disappearance of compound 9. 50 mL of ethyl acetate was added, and the mixture was washed with 100 mL of sodium bicarbonate solution and 50 mL of saturated brine. The organic phase was dried, filtered, and the filtrate was concentrated. The resulting crude product was purified by silica gel column chromatography (gradient elution: petroleum ether/ethyl acetate = 10/1 to 0/1) to obtain pale yellow oily compound 10 (2.44 g, 3.79 mmol, 96% yield).

¹H NMR (400 MHz, DMSO-*d*₆): *δ* 11.16 (br s, 1H), 8.70 (s, 1H), 8.33 (s, 1H), 8.05 (d, *J* = 7.4 Hz, 2H), 7.69-7.61 (m, 1H), 7.60-7.52 (m, 2H), 7.25-7.12 (m, 5H), 7.05 (t, *J* = 8.9 Hz, 4H), 6.78 (dd, *J =* 8.9, 3.1 Hz, 4H), 4.79 (d, *J* = 4.0 Hz, 1H), 4.51-4.28 (m, 2H), 3.81 (d, *J* = 4.0 Hz, 1H), 3.70 (s, 6H), 3.10 (dd, *J* = 9.8, 4.9 Hz, 1H), 2.89 (dd, *J =* 9.7, 5.1 Hz, 1H), 2.37-2.23 (m, 1H), 1.03 (d, *J =* 6.3 Hz, 3H).

LC-MS: *C*₃₈*H*₃₇*N*₅*O*₅, MW 643.2, 644.2 (M+H).

### 1.10 Synthesis of SA000001

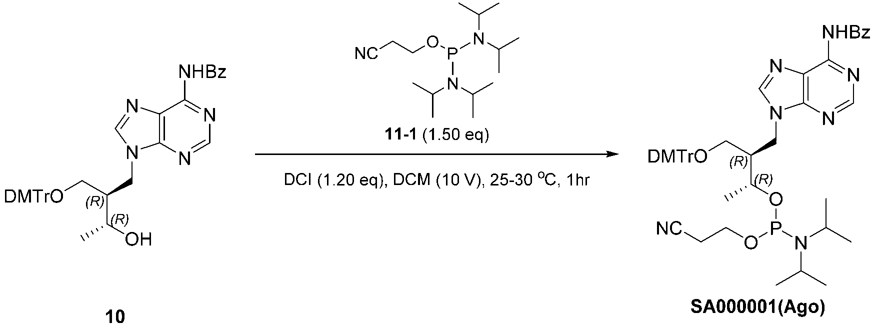

Compound 10 (1.5 g, 2.33 mmol, 1.0 equiv) was placed in a clean, dry reaction flask. Under nitrogen protection, 4 mL of acetonitrile was added, and the acetonitrile was distilled off at 35-40°C. This operation was repeated once to remove water from compound 10. At room temperature, 15 mL of anhydrous dichloromethane was added to the flask. Then, compound 11-1 (1.05 g, 3.5 mmol, 1.11 mL, 1.5 equiv) and 4,5-dicyanoimidazole (358 mg, 3.03 mmol, 1.3 equiv) were added, and the mixture was stirred at room temperature for one hour. TLC indicated the disappearance of compound 10. 30 mL of dichloromethane was added, and the mixture was washed with sodium bicarbonate solution (50 mL x 2) and saturated brine (50 mL). The organic phase was dried, filtered, and the filtrate was concentrated. The resulting crude product was dissolved in 50 mL of methyl tert-butyl ether, and 100 mL of 1% aqueous sodium hydroxide solution was added, followed by stirring for half an hour. The layers were allowed to separate, and the organic phase was collected, washed with saturated brine, dried, filtered, and concentrated. The crude product was purified by C18 reverse-phase column chromatography to obtain pale yellow product SA000001 (1.1 g, 1.3 mmol, 56% yield).

¹H NMR (400 MHz, *CD*₃*CN*): *δ* 9.33 (br s, 1H), 8.61 (br s, 1H), 7.99 (d, *J =* 11.0 Hz, 3H), 7.67-7.60 (m, 1H), 7.59-7.49 (m, 2H), 7.32-6.99 (m, 9H), 6.75 (td, *J =* 9.2, 6.5 Hz, 4H), 4.50-4.38 (m, 1H), 4.36-4.13 (m, 2H), 3.72 (dd, *J* = 4.8, 1.5 Hz, 6H), 3.66-3.45 (m, 3H), 3.25 (td, *J* = 9.6, 5.4 Hz, 1H), 3.01 (ddd, *J* = 17.9, 10.0, 5.8 Hz, 1H), 2.62 (t, *J* = 5.9 Hz, 1H), 2.53 (t, *J =* 5.9 Hz, 1H), 1.32-1.22 (m, 4H), 1.17-1.05 (m, 12H). ³¹P NMR (DMSO-*d*₆, 162 MHz): *δ* ppm 147.7, 146.8.

LCMS: *C*₄₇*H*₅₄*N*₇O₆*P*, MW 843.3, 844.5 (M+H).

### Example 2: Preparation of Compound SA000014

### 2.1 Synthesis of Compound 1-2

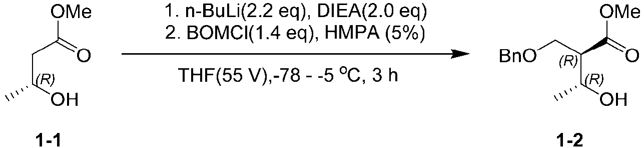

At -78°C under argon protection, a solution of n-butyllithium in tetrahydrofuran (2.5 M, 355.5 mL, 2.1 equiv) was slowly added dropwise to a solution of DIEA (85.7 g, 846.5 mmol, 119.6 mL, 2.0 equiv) in tetrahydrofuran (1.85 L). After the addition was complete, stirring was continued at -78°C for one hour. Subsequently, a solution of compound 1-1 (commercially available from Adamas) (50.0 g, 423.3 mmol, 48.6 mL, 1.0 equiv) in tetrahydrofuran (910.0 mL) was slowly added dropwise to the reaction mixture and stirring was continued for one hour. Then, HMPA (128.0 g, 714.3 mmol, 125.0 mL, 1.69 equiv) and benzyl chloromethyl ether (92.8 g, 592.6 mmol, 81.8 mL, 1.4 equiv) were slowly added dropwise to the reaction mixture, which was then slowly warmed to 0°C and stirred for 3 hours. After the reaction was complete, the reaction mixture was extracted 3 times with ethyl acetate. The combined organic phases were evaporated to dryness, and the resulting crude product was purified by silica gel column chromatography (petroleum ether/ethyl acetate 30:1 to 19:1) to obtain yellow oily compound 1-2 (54.0 g, reaction yield 54%).

¹H NMR data for compound 1-2: (400 MHz, *CDCl*₃) *δ* 7.37-7.27 (m, 5H), 4.53-4.52 (m, 2H), 4.14 (s, 1H), 3.77-3.72 (m, 6H), 2.78-2.74 (m, 1H), 1.24-1.23 (d, *J =* 6.4 Hz, 4H).

Mass spectral identification of compound 1-2 (*C*₁₃*H*₁₈*O*₄, MW 238.1, [M+H] = 239.2).

### 2.2 Synthesis of Compound 1-3

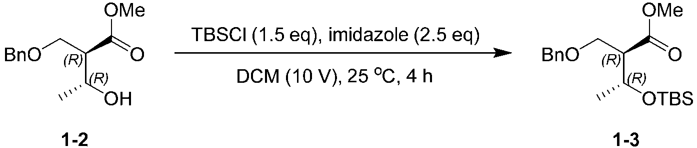

At room temperature, compound 1-2 (51.0 g, 214.0 mmol) was dissolved in dichloromethane (510.0 mL). Subsequently, imidazole (36.4 g, 535.1 mmol, 2.5 equiv) and tert-butyldimethylsilyl chloride (48.4 g, 321.0 mmol, 39.5 mL, 1.5 equiv) were slowly added to the reaction mixture and stirring was continued for 4 hours. After the reaction was complete, the reaction mixture was extracted 3 times with saturated sodium bicarbonate solution and dichloromethane. The combined organic phases were evaporated to dryness, and the resulting crude product was purified by silica gel column chromatography (petroleum ether/ethyl acetate 150:1 to 120:1) to obtain colorless oily compound 1-3 (61.9 g, reaction yield 82%).

¹H NMR data for compound 1-3: (400 MHz, *CDCl*₃) *δ* 7.34-7.27 (m, 5H), 4.55-4.48 (m, 2H), 4.13-4.07 (m, 1H), 3.72-3.67 (m, 4H), 3.60-3.57 (m, 1H), 2.80-2.77 (m, 1H), 1.17-1.59 (d, *J* = 6.0 Hz, 4H), 0.86 (s, 9H), 0.05-0.03 (d, *J* = 8.8 Hz, 6H).

Mass spectral identification of compound 1-3 (*C*₁₉*H*₃₂*O*₄*Si*, MW 352.1, [M+H] = 353.2).

### 2.3 Synthesis of Compound 1-4

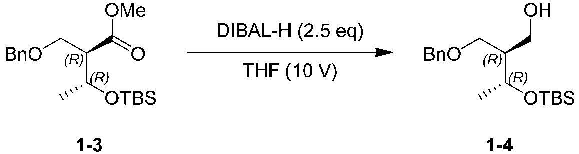

At -78°C, compound 1-3 (60.0 g, 170.2 mmol) was dissolved in tetrahydrofuran (600.0 mL). Subsequently, diisobutylaluminium hydride (1.0 M, 425.5 mL, 2.50 equiv) was slowly added to the reaction mixture and stirring was continued at -78°C for 2 hours. After the reaction was complete, the reaction mixture was extracted 3 times with saturated ammonium chloride solution and ethyl acetate. The combined organic phases were evaporated to dryness, and the resulting crude product was purified by silica gel column chromatography (petroleum ether/ethyl acetate 50:1 to 20:1) to obtain colorless oily compound 1-4 (21.5 g, reaction yield 39%).

¹H NMR data for compound 1-4: (400 MHz, *CDCl*₃) *δ* 7.28-7.19 (m, 5H), 4.46-4.44 (m, 2H), 4.13-4.10 (m, 1H), 3.95-3.91 (m, 1H), 3.67-3.55 (m, 3H), 3.03-3.01 (m, 1H), 1.70-1.63 (m, 1H), 1.17-1.09 (m, 3H), 0.83-0.78 (m, 9H), 0.02 (d, *J* = 5.2 Hz, 6H).

Mass spectral identification of compound 1-4 (*C*₁₈*H*₃₂*O*₃*Si*, MW 324.1, [M+H] = 325.2).

### 2.4 Synthesis of Compound 1-5

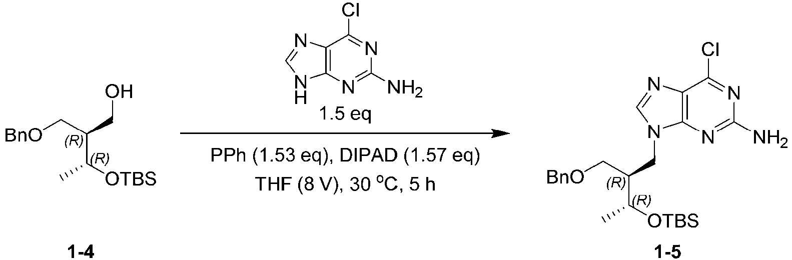

At room temperature under a nitrogen atmosphere, compound 1-4 (5.5 g, 16.9 mmol), 2-amino-6-chloropurine (4.3 g, 25.4 mmol, 1.5 equiv) and triphenylphosphine (6.8 g, 25.9 mmol, 1.53 equiv) were dissolved in tetrahydrofuran (93.0 mL). Subsequently, under an ice-water bath, diisopropyl azodicarboxylate (5.4 g, 26.6 mmol, 5.16 mL, 1.57 equiv) was slowly added to the reaction mixture. After the addition was complete, the reaction mixture was stirred at room temperature for 5 hours. After the reaction was complete, the reaction mixture was extracted 3 times with saturated ammonium chloride solution and ethyl acetate. The combined organic phases were evaporated to dryness, and the resulting crude product was purified by silica gel column chromatography (petroleum ether/ethyl acetate 10:1 to 3:1) to obtain yellow oily compound 1-5 (7.6 g, reaction yield 95%).

¹H NMR data for compound 1-5: (400 MHz, *CDCl*₃) δ 7.51 (s, 1H), 7.16-7.08 (m, 5H), 4.99 (s, 2H), 4.83-4.77 (m, 7H), 4.26-4.12 (m, 2H), 3.97-3.85 (m, 3H), 3.67-3.55 (m, 3H), 3.23-3.09 (m, 2H), 2.05-2.00 (m, 1H), 1.87 (s, 2H), 1.09 (d, *J =* 6.4 Hz, 19H), 1.01 (d, *J* = 6.4 Hz, 3H), 0.74-0.70 (m, 9H), 0.01 (d, *J =* 14.0 Hz, 6H).

Mass spectral identification of compound 1-5 (*C*_{*2*3}*H*₃₄*ClN*₅*O*₂*Si*, MW 475.1, [M+H] = 476.2).

### 2.5 Synthesis of Compound 1-6

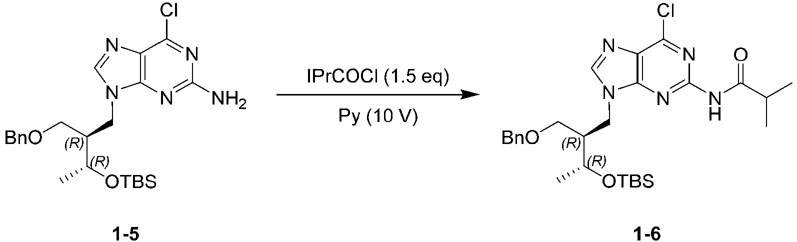

At room temperature, compound 1-5 (11.0 g, 23.1 mmol) was dissolved in pyridine (110.0 mL). Isobutyryl chloride (3.69 g, 34.66 mmol, 3.63 mL, 1.5 equiv) was slowly added to the reaction mixture, and the mixture was then stirred at room temperature for 3 hours. After the reaction was complete, the solvent pyridine was removed by vacuum distillation. The remaining system was extracted 3 times with saturated sodium bicarbonate solution and ethyl acetate. The combined organic phases were evaporated to dryness to obtain crude light brown oily compound 1-6 (22.0 g), which was used directly in the next step.

Mass spectral identification of compound 1-6 (*C*₂₇*H*₄₀*ClN*₅*O*₃*Si*, MW 545.1, [M+H] = 546.3).

### 2.6 Synthesis of Compound 1-7

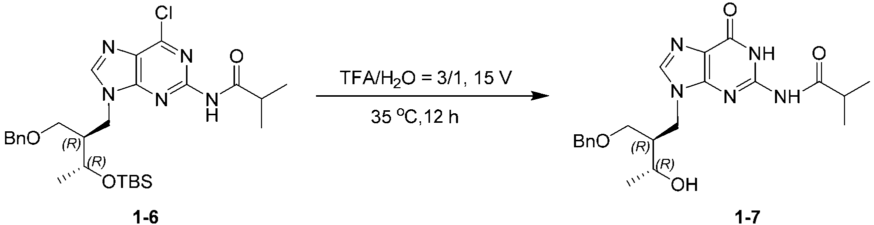

At room temperature, compound 1-6 (10.0 g, 18.3 mmol) was dissolved in trifluoroacetic acid (111.1 mL) and water (35.0 mL). The reaction mixture was stirred at 35°C for 12 hours. After the reaction was complete, the water and trifluoroacetic acid were removed by vacuum distillation. The remaining system was extracted 3 times with saturated sodium bicarbonate solution and ethyl acetate. The combined organic phases were evaporated to dryness, and the resulting crude product was purified by silica gel column chromatography (dichloromethane/methanol 100:1 to 25:1) to obtain white solid compound 1-7 (4.8 g, reaction yield 27%).

¹H NMR data for compound 1-7: (400 MHz, *CDCl*₃) *δ* 12.0 (s, 1H), 11.6 (s, 1H), 7.91 (s, 1H), 7.28-7.18 (m, 5H), 4.80 (d, *J =* 4.40 Hz, 1H), 4.35-4.23 (m, 3H), 4.15-4.09 (m, 1H), 3.77-3.73 (m, 1H), 3.32-3.27 (m, 2H), 3.23-3.09 (m, 2H), 2.83-2.76 (m, 1H), 2.18-2.15(m, 1H), 1.13-1.10 (m, 9H).

Mass spectral identification of compound 1-7 (*C*₂₁*H*₂₇*N*₅*O*₄, MW 413.1, [M+H] = 414.2).

### 2.7 Synthesis of Compound 1-8

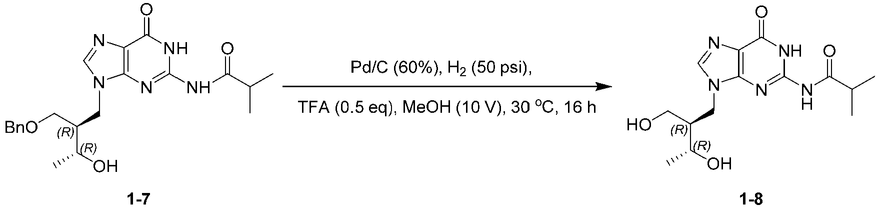

At room temperature under hydrogen (50 psi), compound 1-7 (4.3 g, 10.4 mmol) was dissolved in methanol (43.0 mL). Trifluoroacetic acid (592.9 mg, 5.2 mmol, 0.38 mL, 0.5 equiv) and palladium on carbon (2.69 g, 2.53 mmol, 2.43 equiv) were added to the reaction system, respectively. The reaction mixture was then stirred at 30°C for 16 hours. After the reaction was complete, the palladium on carbon was removed by filtration. The remaining system was subjected to vacuum distillation to obtain the crude product, white solid compound 1-8 (3.0 g, reaction yield 87%).

¹H NMR data for compound 1-8: (400 MHz, DMSO-*d*₆) δ 12.1 (s, 1H), 11.6 (s, 1H), 7.95 (s, 1H), 4.26-4.05 (m, 2H), 3.74-3.71 (m, 1H), 3.29 (d, *J =* 4.8 Hz, 2H), 2.82-2.75 (m, 1H), 1.94-1.90 (m, 1H), 1.13-1.10 (m, 9H).

Mass spectral identification of compound 1-8 (*C*₁₄*H*₂₁*N*₅*O*₄, MW 323.1, [M+H] = 324.2).

### 2.8 Synthesis of Compound 1-9

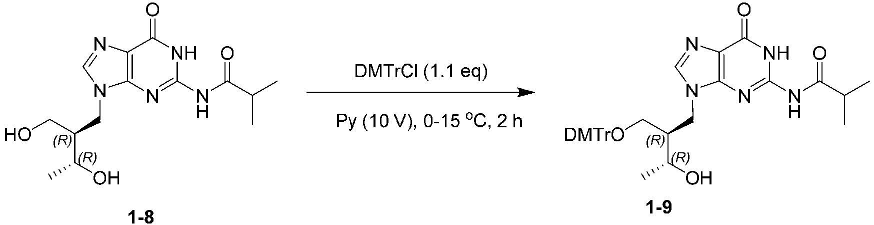

At room temperature, compound 1-8 (3.0 g, 9.28 mmol) was dissolved in pyridine (30.0 mL). Under an ice-water bath, 4,4'-dimethoxytrityl chloride (3.46 g, 10.2 mmol, 1.1 equiv) was added to the reaction system. The reaction mixture was then stirred at 15°C for 2 hours. After the reaction was complete, the solvent pyridine was removed by vacuum distillation. The remaining system was extracted 3 times with saturated sodium bicarbonate solution and ethyl acetate. The combined organic phases were evaporated to dryness, and the resulting crude product was purified by silica gel column chromatography (petroleum ether/ethyl acetate 3:1 to 0:1) to obtain white solid compound 1-9 (3.5 g, reaction yield 59%).

¹H NMR data for compound 1-9: (400 MHz, DMSO-*d*₆) *δ* 12.0 (s, 1H), 11.5 (s, 1H), 7.87 (s, 1H), 7.22-7.14 (m, 5H), 7.04 (t, *J =* 8.8 Hz, 4H), 6.78-6.74 (m, 4H), 4.75 (d, *J* = 3.6 Hz, 1H), 4.25-4.21 (m, 1H), 4.09-4.01 (m, 1H), 3.71 (d, *J =* 1.2 Hz, 1H), 3.07-3.04 (m, 1H), 2.24-2.22 (m, 1H), 1.13-1.06 (m, 9H).

Mass spectral identification of compound 1-9 (*C*₃₅*H*₃₉*N*₅*O*₆, MW 625.1, [M+H] = 626.4).

### 2.9 Synthesis of Compound SA000014

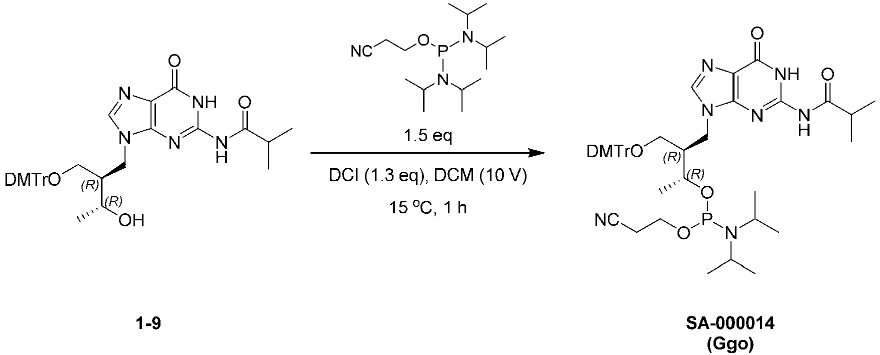

At room temperature under a nitrogen atmosphere, compound 1-9 (3.0 g, 4.79 mmol) was dissolved in dry acetonitrile (4.0 mL). The acetonitrile was then removed by vacuum distillation, and this process was repeated three times. The remaining system was dissolved in dichloromethane (30.0 mL). 4,5-dicyanoimidazole (736.1 mg, 6.23 mmol, 1.3 equiv) and bis(diisopropylamino)(2-cyanoethoxy)phosphine (2.17 g, 7.19 mmol, 2.28 mL, 1.5 equiv) were added to the reaction system, respectively. The reaction mixture was then stirred at 15°C for 1 hour. After the reaction was complete, the reaction mixture was dissolved in dichloromethane (12.0 mL) and 1% sodium hydroxide solution (40.0 mL), and stirring was continued at 15°C for 0.5 hours. The crude reaction product was purified by C18 reverse-phase column chromatography to obtain white solid compound SA000014 (2.2 g, reaction yield 53%).

¹H NMR data for compound SA000014: (400 MHz, *CD*₃*CN*) δ 11.86 (s, 1H), 9.58 (s, 1H), 7.58-7.54 (m, 1H), 7.29-7.15 (m, 9H), 6.76 (s, 4H), 4.24-4.05 (m, 3H), 3.73-3.52 (m, 10H), 3.24-3.22 (m, 1H), 3.02-2.94 (m, 1H), 2.86-2.72 (m, 4H), 1.25-1.03 (m, 23H). ³¹P NMR data: (400 MHz, *CD*₃*CN*) *δ* 147.6, 146.8.

Mass spectral identification of compound SA000014 (*C*₄₄*H*₅₆*N*₇*O*₇*P*, MW 825.1, [M+H] = 826.5).

### Example 3: Preparation of Compound SA000015

### 3.1 Synthesis of Compound C-1

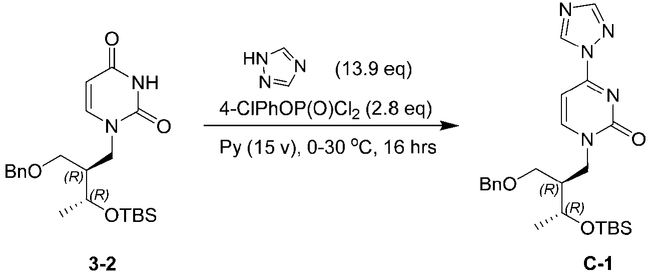

At room temperature, compound 3-2 (8.5 g, 20.3 mmol, prepared in Example 5) and 1,2,4-triazole (19.5 g, 282.0 mmol, 13.9 equiv) were dissolved in pyridine (128.0 mL). Subsequently, under an ice-water bath, 4-chlorophenyl dichlorophosphate (56.9 mmol, 9.25 mL, 2.8 equiv) was added dropwise to the reaction system. The reaction mixture was stirred at 30°C for 16 hours. After the reaction was complete, the solvent pyridine was removed by vacuum distillation. The remaining system was extracted 3 times with water and ethyl acetate. The combined organic phases were evaporated to dryness to obtain the crude yellow oily product compound C-1 (9.5 g), which was used directly in the next step. Mass spectral identification of compound C-1 (*C*₂₄*H*₃₅*N*₅*O*₃*Si*, MW 469.1, [M+H] = 470.3).

### 3.2 Synthesis of Compound C-2

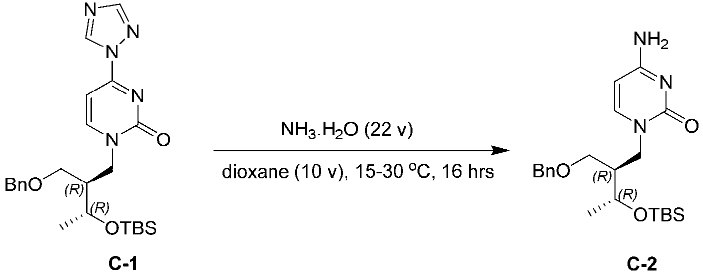

At room temperature, compound C-1 (9.5 g, 20.2 mmol) was dissolved in 1,4-dioxane (95.0 mL). Subsequently, aqueous ammonia (1.38 mol, 212.1 mL, 25% purity, 68.0 equiv) was added to the reaction system. The reaction mixture was stirred at 30°C for 16 hours. After the reaction was complete, the solvent was removed by vacuum distillation. The remaining system was extracted 3 times with water and dichloromethane. The combined organic phases were evaporated to dryness to obtain the crude yellow solid product compound C-2 (8.5 g), which was used directly in the next step.

Mass spectral identification of compound C-2 (*C*₂₂*H*₃₅*N*₃*O*₃*Si*, MW 417.1, [M+H] = 418.2).

### 3.3 Synthesis of Compound C-3

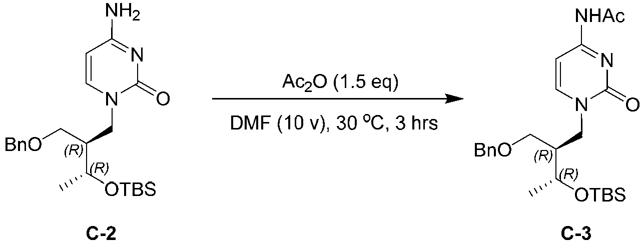

At room temperature, compound C-2 (8.5 g, 20.2 mmol) was dissolved in N,N-dimethylformamide (85.0 mL). Subsequently, acetic anhydride (3.1 g, 30.4 mmol, 2.85 mL, 1.5 equiv) was slowly added to the reaction system. The reaction mixture was stirred at 30°C for 3 hours. After the reaction was complete, the solvent was removed by vacuum distillation. The remaining system was extracted 3 times with saturated sodium bicarbonate solution and ethyl acetate. The combined organic phases were evaporated to dryness, and the resulting crude product was purified by silica gel column chromatography (dichloromethane/methanol 1:0 to 100:1) to obtain yellow solid compound C-3 (4.2 g, reaction yield 43%).

¹H NMR data for compound C-3: (400 MHz, DMSO-*d*₆) *δ* 10.7 (s, 1H), 7.79 (d, *J* = 7.2 Hz, 1H), 7.29 (m, 5H), 7.08 (d, *J =* 7.2 Hz, 1H), 4.36 (d, *J =* 1.2 Hz, 2H), 4.05-3.99 (m, 2H), 3.72-3.67 (m, 1H), 3.39 (dd, *J* = 6.4 Hz, 2H), 2.15-2.08 (m, 1H), 2.08 (s, 3H), 1.15 (d, *J* = 6.4 Hz, 3H), 0.85 (s, 9H), 0.02 (d, *J* = 6.8 Hz, 6H).

Mass spectral identification of compound C-3 (*C*₂₄*H*₃₇*N*₃*O*₄*Si*, MW 459.1, [M+H] = 460.2).

### 3.4 Synthesis of Compound C-4

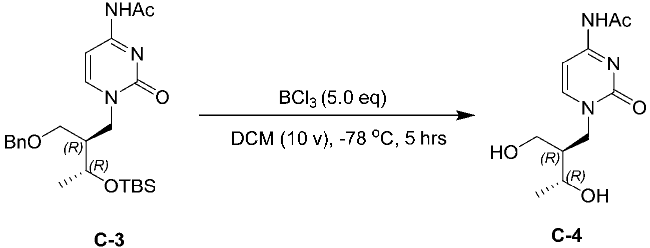

At -78°C, compound C-3 (4.3 g, 9.35 mmol) was dissolved in dichloromethane (46.0 mL). Subsequently, boron trichloride (1.0 M in DCM, 46.8 mL, 5.0 equiv) was slowly added to the reaction system. The reaction mixture was then stirred at -78°C for 4 hours. After the reaction was complete, the reaction was quenched with triethylamine (40.0 mL) and methanol (88.0 mL). The remaining system was extracted 3 times with water and dichloromethane. The combined organic phases were evaporated to dryness, and the resulting crude product was purified by C18 reverse-phase column chromatography to obtain white solid compound C-4 (0.96 g, reaction yield 40%).

¹H NMR data for compound C-4: (400 MHz, DMSO-*d*₆) δ 10.7 (s, 1H), 8.01 (d, *J* = 7.2 Hz, 1H), 7.12 (d, *J =* 7.2 Hz, 1H), 4.60 (d, *J =* 4.8 Hz, 2H), 4.53-4.50 (m, 1H), 4.00-3.99 (m, 1H), 3.75-3.70 (m, 2H), 3.37-3.32 (m, 2H), 2.08 (s, 3H), 1.81-1.77 (m, 1H), 1.01 (d, *J* = 6.4 Hz, 3H).

Mass spectral identification of compound C-4 (*C*₁₁*H*₁₇*N*₃*O*₄, MW 255.1, [M+H] = 256.2).

### 3.5 Synthesis of Compound C-5

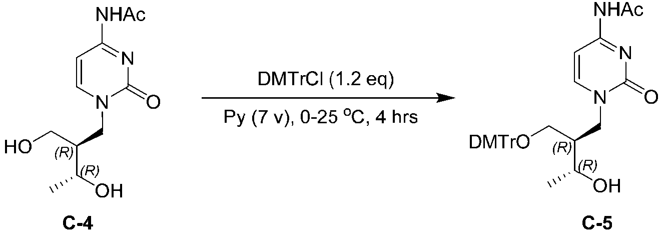

At room temperature, compound C-4 (1.5 g, 5.88 mmol) was dissolved in pyridine (10.0 mL). Under an ice-water bath, 4,4'-dimethoxytrityl chloride (2.4 g, 7.05 mmol, 1.2 equiv) was added to the reaction system. The reaction mixture was then stirred at room temperature for 4 hours. After the reaction was complete, the solvent pyridine was removed by vacuum distillation. The remaining system was extracted 3 times with saturated sodium bicarbonate solution and ethyl acetate. The combined organic phases were evaporated to dryness, and the resulting crude product was purified by silica gel column chromatography (petroleum ether/ethyl acetate 4:1 to 0:1) to obtain white solid compound C-5 (0.7 g, reaction yield 70%).

¹H NMR data for compound C-5: (400 MHz, *CDCl*₃) *δ* 9.71 (s, 1H), 7.39 (d, *J =* 7.3 Hz, 3H), 7.35-7.21 (m, 7H), 7.06 (d, *J* = 7.1 Hz, 1H), 6.85 (d, *J* = 8.8 Hz, 4H), 4.71 (d, *J =* 3.3 Hz, 1H), 4.43 (dd, *J =* 13.8, 4.5 Hz, 1H), 4.23-3.99 (m, 2H), 3.80 (s, 6H), 3.50-3.38 (m, 1H), 3.31 (dd, *J =* 10.0, 4.1 Hz, 1H), 2.69 (t, *J* = 9.4 Hz, 1H), 2.21 (s, 3H), 1.08 (d, *J* = 6.1 Hz, 3H).

Mass spectral identification of compound C-5 (*C*₃₂*H*₃₅*N*₃*O*₆, MW 557.1, [M+H] = 558.4).

### 3.6 Synthesis of Compound SA000015

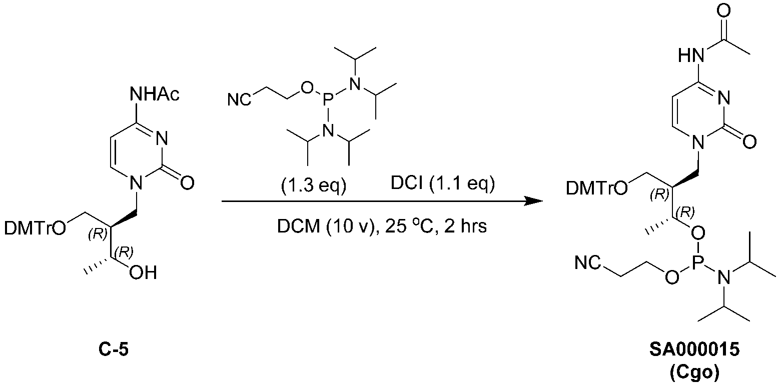

At room temperature under a nitrogen atmosphere, compound C-5 (2.0 g, 3.59 mmol) was dissolved in dichloromethane (20.0 mL). 4,5-dicyanoimidazole (466.0 mg, 3.95 mmol, 1.1 equiv) and bis(diisopropylamino)(2-cyanoethoxy)phosphine (1.4 g, 4.66 mmol, 1.48 mL, 1.3 equiv) were added to the reaction system, respectively. The reaction mixture was then stirred at room temperature for 2 hours. After the reaction was complete, the reaction mixture was dissolved in methyl tert-butyl ether (10.0 mL) and 1% sodium hydroxide solution (10.0 mL), and stirring was continued at room temperature for 0.5 hours. The remaining system was extracted 3 times with saturated sodium bicarbonate solution and dichloromethane. The combined organic phases were evaporated to dryness, and the resulting crude product was purified by C18 reverse-phase column chromatography to obtain white solid compound SA000015 (1.5 g, reaction yield 55%).

¹H NMR data for compound SA000015: (400 MHz, *CD*₃*CN*) *δ* 9.15 (s, 1H), 7.50-7.18 (m, 11H), 6.84-6.80 (m, 4H), 4.22-4.08 (m, 2H), 3.75-3.22 (m, 11H), 3.23-3.22 (m, 2H), 2.62-2.51 (m, 2H), 2.38-2.35 (m, 1H), 2.12 (s, 3H), 1.022-1.07 (m, 15H). ³¹P NMR data: (400 MHz, *CD*₃*CN*) *δ* 147.5, 146.7.

Mass spectral identification of compound SA000015 (*C*₄₁*H*₅₂*N*₅*O*₇*P*, MW 757.1, [M+H] = 758.5).

### Example 4: Preparation of Compound SA000016

### 4.1 Synthesis of Compound 3-1

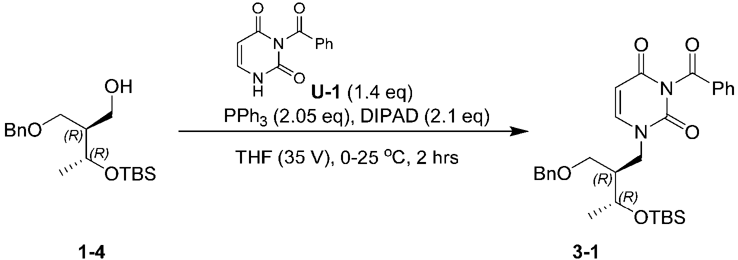

Under an ice-water bath, compound 1-4 (9.99 g, 30.8 mmol), compound U-1 (commercially available from Shanghai Haohong Biomedical Technology Co., Ltd.) (9.33 g, 43.1 mmol, 1.4 equiv), and triphenylphosphine (16.6 g, 63.2 mmol, 2.0 equiv) were dissolved in dry tetrahydrofuran (350.0 mL), respectively. Subsequently, diisopropyl azodicarboxylate (13.1 g, 64.7 mmol, 12.6 mL, 2.1 equiv) was added dropwise to the reaction system. The reaction mixture was then stirred at room temperature for 2 hours. After the reaction was complete, the reaction mixture was extracted 3 times with saturated sodium bicarbonate solution and ethyl acetate. The combined organic phases were evaporated to dryness, and the resulting crude product was purified by silica gel column chromatography (petroleum ether/ethyl acetate 10: 1 to 8:1) to obtain white solid compound 3-1 (16.1 g, reaction yield 99%).

¹H NMR data for compound 3-1: (400 MHz, DMSO-*d*₆) *δ* 7.89-7.74 (m, 4H), 7.56-7.53 (m, 2H), 7.33-7.29 (m, 5H), 5.80-5.73 (m, 1H), 4.46-4.38 (m, 2H), 4.00-3.91 (m, 2H), 3.60-3.50 (m, 1H), 3.49-3.43 (m, 2H) 2.17-1.98 (m, 1H), 1.13 (s, 3H), 0.83 (s, 9H), 0.01 (d, *J* = 6.0 Hz, 6H).

Mass spectral identification of compound 3-1 (*C*₂₉*H*₃₈*N*₂*O*₅*Si*, MW 522.1, [M+H] = 523.2).

### 4.2 Synthesis of Compound 3-2

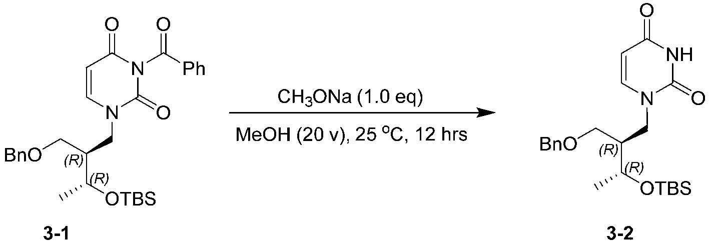

At room temperature, compound 3-1 (8.05 g, 15.4 mmol) was dissolved in methanol (160.0 mL). Subsequently, sodium methoxide (2.77 g, 15.4 mmol, 30% purity, 1.0 equiv) was slowly added to the reaction system. The reaction mixture was then stirred at room temperature for 12 hours. After the reaction was complete, the solvent methanol was removed by vacuum distillation. The remaining system was extracted 3 times with 1M hydrochloric acid solution and ethyl acetate. The combined organic phases were evaporated to dryness, and the resulting crude product was purified by silica gel column chromatography (petroleum ether/ethyl acetate 10:1 to 0:1) to obtain white solid compound 3-2 (6.1 g, reaction yield 94%).

¹H NMR data for compound 3-2: (400 MHz, DMSO-*d*₆) δ 11.1 (s, 1H), 7.48 (d, *J* = 8.0 Hz, 1H), 7.30-7.24 (m, 5H), 5.47-5.44 (m, 1H), 4.45-4.35 (m, 2H), 3.98-3.94 (m, 1H), 3.79-3.78 (m, 1H), 3.63-3.57 (m, 1H), 3.38-3.35 (m, 2H), 2.04-1.95 (m, 1H), 1.10 (s, 3H), 0.83 (s, 9H), 0.01 (d, *J* = 6.0 Hz, 6H).

Mass spectral identification of compound 3-2 (*C*₂₂*H*₃₄*N*₂*O*₄*Si*, MW 418.1, [M+H] = 418.2).

### 4.3 Synthesis of Compound 3-3

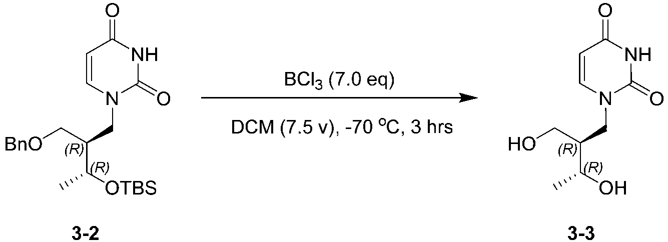

At -70°C, compound 3-2 (4.0 g, 9.56 mmol) was dissolved in dichloromethane (30.0 mL). Subsequently, boron trichloride (1.0 M in DCM, 66.9 mL, 7.0 equiv) was slowly added to the reaction system. The reaction mixture was then stirred at -70°C for 3 hours. After the reaction was complete, the reaction was quenched with triethylamine (5.0 mL) and methanol (30.0 mL). The remaining system was extracted 3 times with water and dichloromethane. The combined organic phases were evaporated to dryness, and the resulting crude product was purified by C18 reverse-phase column chromatography to obtain white solid compound 3-3 (0.7 g, reaction yield 33%).

¹H NMR data for compound 3-3: (400 MHz, DMSO-*d*₆) *δ* 7.54 (d, *J* = 8.0 Hz, 1H), 5.51 (d, *J=* 7.6 Hz, 1H), 3.88-3.84 (m, 1H), 3.72-3.70 (m, 1H), 3.61-3.59 (m, 2H), 1.75-1.68 (m, 1H), 1.09 (d, *J* = 6.4 Hz, 3H).

Mass spectral identification of compound 3-3 (*C*₉*H*₁₄*N*₂*O*₄, MW 214.1, [M+H] = 215.2).

### 4.4 Synthesis of Compound 3-4

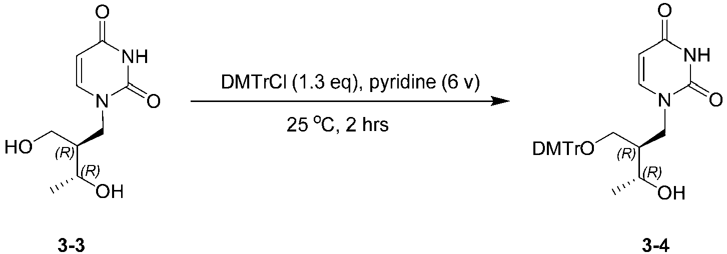

At room temperature, compound 3-1 (0.4 g, 1.87 mmol) was dissolved in pyridine (4.0 mL). Under an ice-water bath, 4,4'-dimethoxytrityl chloride (949.0 mg, 2.8 mmol, 1.5 equiv) was added to the reaction system. The reaction mixture was then stirred at room temperature for 2 hours. After the reaction was complete, the solvent pyridine was removed by vacuum distillation. The remaining system was extracted 3 times with saturated sodium bicarbonate solution and ethyl acetate. The combined organic phases were evaporated to dryness, and the resulting crude product was purified by silica gel column chromatography (petroleum ether/ethyl acetate 4:1 to 0:1) to obtain white solid compound 3-4 (0.7 g, reaction yield 70%).

¹H NMR data for compound 3-4: (400 MHz, DMSO-*d*₆) δ 11.3 (s, 1H), 7.37 (d, *J =* 8.0 Hz, 1H), 7.33-7.29 (m, 4H), 7.20-7.17 (m, 5H), 6.86-6.83 (m, 4H), 5.40 (d, *J =* 8.0 Hz, 1H), 4.61 (d, *J* = 4.8 Hz, 2H), 3.83-3.79 (m, 2H), 3.72 (s, 6H), 3.71-3.68 (m, 1H), 3.08-2.90 (m, 2H), 1.90 (s, 1H), 1.01 (d, *J =* 6.4 Hz, 3H).

Mass spectral identification of compound 3-4 (*C*₃₀*H*₃₂*N*₂*O*₆, MW 516.1, [M+H] = 517.4).

### 4.5 Synthesis of Compound SA000016

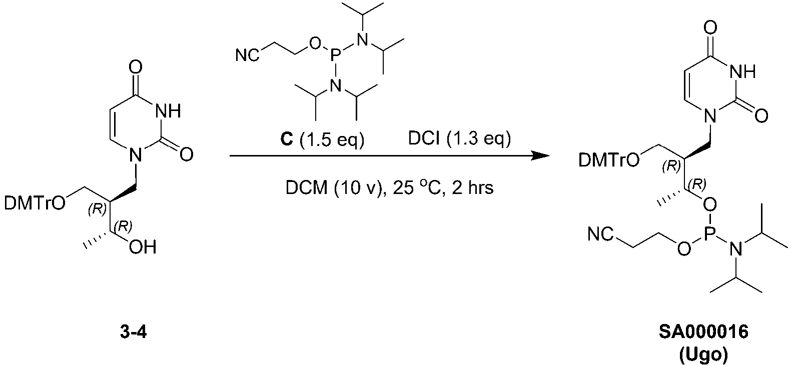

At room temperature under a nitrogen atmosphere, compound 3-4 (1.2 g, 2.32 mmol) was dissolved in dichloromethane (12.0 mL). 4,5-dicyanoimidazole (357.0 mg, 3.02 mmol, 1.3 equiv) and bis(diisopropylamino)(2-cyanoethoxy)phosphine (1.05 g, 3.48 mmol, 1.5 equiv) were added to the reaction system, respectively. The reaction mixture was then stirred at room temperature for 2 hours. After the reaction was complete, the reaction mixture was dissolved in methyl tert-butyl ether (10.0 mL) and 1% sodium hydroxide solution (10.0 mL), and stirring was continued at room temperature for 0.5 hours. The remaining system was extracted 3 times with ethyl acetate. The combined organic phases were evaporated to dryness, and the resulting crude product was purified by C18 reverse-phase column chromatography to obtain white solid compound SA000016 (1.2 g, reaction yield 71%).

¹H NMR data for compound SA000016: (400 MHz, *CD*₃*CN*) *δ* 9.39 (s, 1H), 7.39-7.24 (m, 10H), 6.85-6.81 (m, 4H), 5.43-5.39 (m, 1H), 4.21-4.18 (m, 1H), 3.86-3.77 (m, 1H), 3.75 (s, 6H), 3.56-3.53 (m, 5H), 3.28-3.05 (m, 2H), 2.62-2.61 (m, 2H), 2.22-2.19 (m, 2H), 1.22-1.07 (m, 15H). ³¹P NMR data: (400 MHz, *CD*₃*CN*) *δ* 147.6, 146.5.

Mass spectral identification of compound SA000016 (*C*₃₉*H*₄₉*N*₄*O*₇*P*, MW 716.1, [M+H] = 717.5).

### 4.6 Synthesis of Tgo Monomer

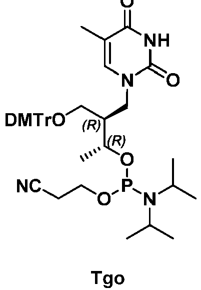

According to the synthesis method for Ugo in the example, white solid Tgo was prepared (4.8 g, 6.6 mmol, 74% yield). Molecular formula of compound Tgo: *C*₄₀*H*₅₁*O*₇*N*₄*P*, MW: 730.3, LC-MS found 753.4 (M+Na). ¹H NMR (400 MHz, DMSO-*d*₆): δ 11.75 (d, *J* = 215.2 Hz, 1H), 7.90 (d, *J* = 4.6 Hz, 1H), 7.23 - 7.22 (m, 4H), 7.20 - 7.14 (m, 1H), 7.09

(td, *J =* 7.1, 3.5 Hz, 4H), 7.23 - 7.22 (m, 4H), 4.23 (dd, *J* = 13.9, 5.2 Hz, 1H), 4.14 - 3.99 (m, 2H), 3.71 (s, 6H), 3.59 - 3.53 (m, 1H), 3.51 - 3.43 (m, 2H), 3.13 (td, *J* = 9.4, 5.5 Hz, 1H), 3.05 - 2.93 (m, 1H), 2.82 (dt, *J* = 13.5, 6.8 Hz, 1H), 2.73 (t, *J* = 5.9 Hz, 1H), 2.65 (t, *J* = 5.8 Hz, 1H), 1.26 - 0.98 (m, 19H). ³¹P NMR (162 MHz, DMSO-*d*₆): δ 147.0, 146.6.

### Example 5: Preparation of Compound SA000068 (Ggs)

### 5.1 Preparation of Intermediate 1-2

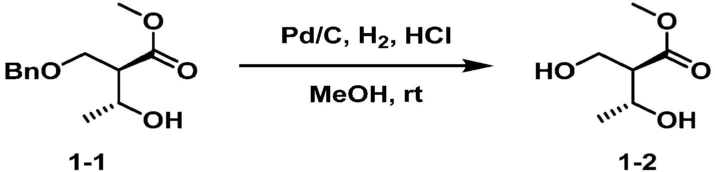

Compound 1-1 (45.7 mmol, 10.9 g) was placed in a clean, dry reaction flask. 110 mL of methanol was added. At room temperature under a hydrogen atmosphere, palladium on carbon (wet basis, 10% Pd/C) (22% wt, 2.4 g) was added. The mixture was then stirred at room temperature for 16 hours. After the reaction, the palladium on carbon was removed by filtration, and the filtrate was concentrated to obtain the crude oily product compound 1-2 (6.8 g, 45.7 mmol, 100% yield), which was used directly in the next step without purification. Molecular formula of compound 1-2: *C*₆*H*₁₂*O*₄, MW: 148.07, LC-MS measured: 149.2 (M+H).

### 5.2 Preparation of Intermediate 1-3

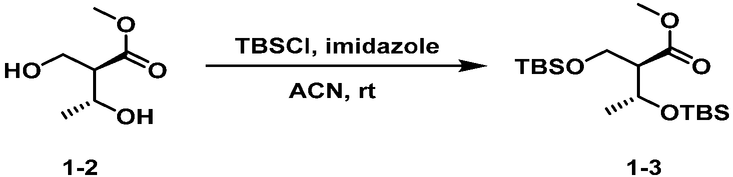

Compound 1-2 (45.7 mmol, 6.8 g) was placed in a clean, dry reaction flask. 70 mL of acetonitrile and imidazole (6.0 equiv, 274.2 mmol, 18.67 g) were added. Under an ice-water bath, TBSCl (3.0 equiv, 137.1 mmol, 20.66 g) was added slowly in batches. The mixture was then stirred at room temperature for 16 hours. After the reaction, most of the solvent was removed by concentration. The residue was dissolved in 200 mL of ethyl acetate, washed with 200 mL of water, dried, and evaporated to dryness. The resulting crude product was purified by silica gel column chromatography (gradient elution: petroleum ether/ethyl acetate = 100/0 - 100/5) to obtain pale yellow oily compound 1-3 (15.8 g, 41.9 mmol, 92% yield). Molecular formula of compound 1-3: *C*₁₈*H*₄₀*O*₄*Si*₂, MW: 376.25, LC-MS measured: 377.3 (M+H).

### 5.3 Preparation of Intermediate 1-4

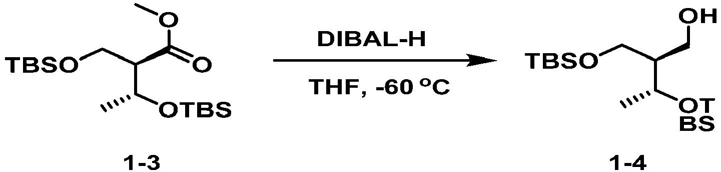

Under an argon atmosphere, compound 1-3 (41.9 mmol, 15.8 g) was placed in a clean, dry reaction flask, dissolved in 158 mL of tetrahydrofuran, and cooled to -78°C. DIBAL-H (2.2 equiv, 92.3 mmol, 92.3 mL) was added dropwise while maintaining the temperature below -60°C. The temperature was controlled between -70°C and -60°C until the reaction was complete. The reaction was quenched by dropwise addition of saturated ammonium chloride. 100 mL of ethyl acetate and 200 mL of potassium sodium tartrate solution were added, and the mixture was stirred for 30 min. The organic phase was dried, filtered, and concentrated. The resulting crude product was purified by silica gel column chromatography (gradient elution: petroleum ether/ethyl acetate = 100/0 - 50/50) to obtain pale yellow oily compound 1-4 (13.1 g, 37.4 mmol, 89% yield). Molecular formula of compound 1-4: *C*₁₇*H*₄₀*O*₃*Si*₂, MW: 348.25, LC-MS measured: 349.3 (M+H).

### 5.4 Preparation of Intermediate 1-5

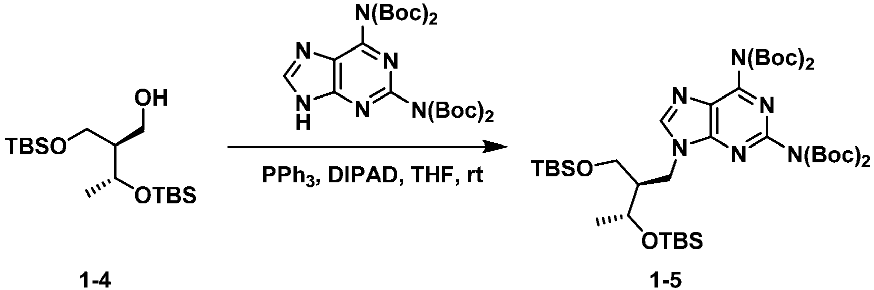

Compound 1-4 (37.3 mmol, 13.0 g) was placed in a clean, dry reaction flask. 130 mL of tetrahydrofuran was added. At room temperature, the purine intermediate (1.1 equiv, 41.0 mmol, 22.6 g) and triphenylphosphine (1.5 equiv, 55.9 mmol, 11.3 g) were added. The mixture was cooled to below 0°C, and DIPAD (1.5 equiv, 55.9 mmol, 14.7 g) was added dropwise. The mixture was then stirred at room temperature for 2 hours. After the reaction, the reaction mixture was concentrated. The resulting crude product was purified by silica gel column chromatography (gradient elution: petroleum ether/ethyl acetate = 100/0 - 30/70) to obtain pale yellow oily compound 1-5 (32.3 g, 36.6 mmol, 98% yield). Molecular formula of compound 1-5: *C*₄₂*H*₇₆*N*₆*O*₁₀*Si*₂, MW: 880.52, LC-MS measured: 881.7 (M+H).

### 5.5 Preparation of Intermediate 1-6

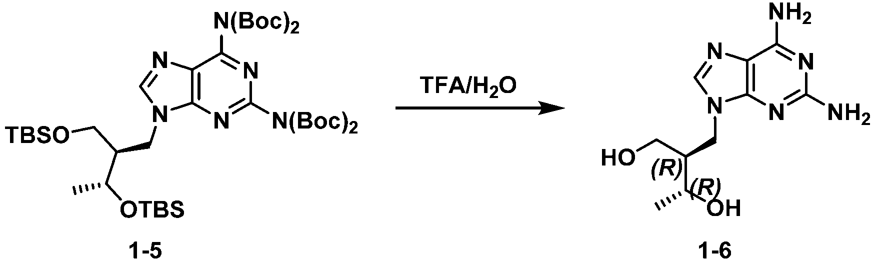

Compound 1-5 (25.0 mmol, 22.0 g) was placed in a clean, dry reaction flask. 66 mL of trifluoroacetic acid and 22 mL of water were added, and the mixture was stirred at room temperature for 2 h. The solvent was removed by concentration. 30 mL of acetonitrile and 30 mL of water were added, and the mixture was stirred for 2 h, filtered, and dried to obtain the target product 1-6 as a white solid (6.3 g, 17.2 mmol, 69% yield). Molecular formula of compound 1-6: *C*₁₀*H*₁₆*N*₆*O*₂, MW: 252.13, LC-MS measured: 253.2 (M+H).

### 5.6 Preparation of Intermediate 1-7

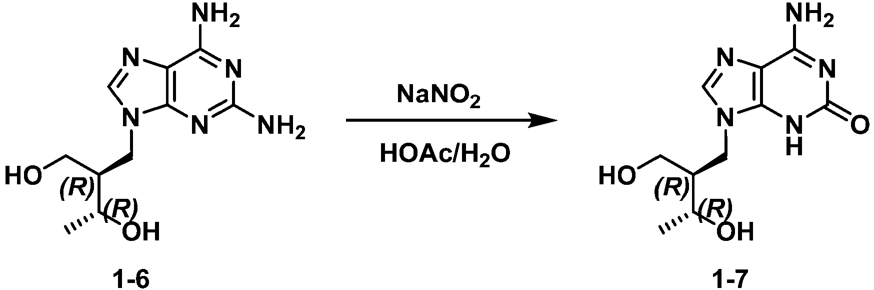

Compound 1-6 (17.2 mmol, 6.3 g) was dissolved in 120 mL of water and heated to 50°C. A solution of sodium nitrite (3.0 equiv, 51.6 mmol, 3.56 g) in 30 mL of water was added dropwise to the mixture, followed by the dropwise addition of acetic acid (7.0 equiv, 120.4 mmol, 7.2 g). The mixture was stirred at this temperature for 10 min. After cooling, aqueous ammonia was added dropwise to adjust the pH to 8. The mixture was concentrated, and the resulting crude product was purified by C18 reverse-phase column chromatography to obtain white solid 1-7 (4.2 g, 16.6 mmol, 66% yield). Molecular formula of compound 1-7: *C*₁₀*H*₁₅*N*₅*O*₃, MW: 253.12, LC-MS measured: 254.2 (M+H).

### 5.7 Preparation of Intermediate 1-8

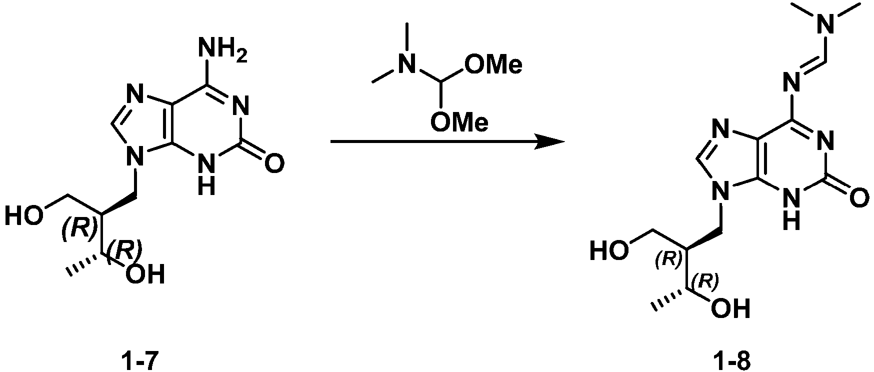

Compound 1-7 (25.3 mmol, 6.4 g) was placed in a clean, dry reaction flask. 128 mL of methanol and N,N-dimethylformamide dimethyl acetal (3.0 equiv, 75.9 mmol, 9.1 g) were added. The mixture was stirred at room temperature overnight. After the reaction, the mixture was directly concentrated to obtain an off-white powder 1-8 (7.6 g, 24.6 mmol, 98% yield), which was used directly in the next step without purification. Molecular formula of compound 1-8: *C*₁₃*H*₂₀*N*₆*O*₃, MW: 308.16, LC-MS measured: 309.2 (M+H).

### 5.8 Preparation of Intermediate 1-9

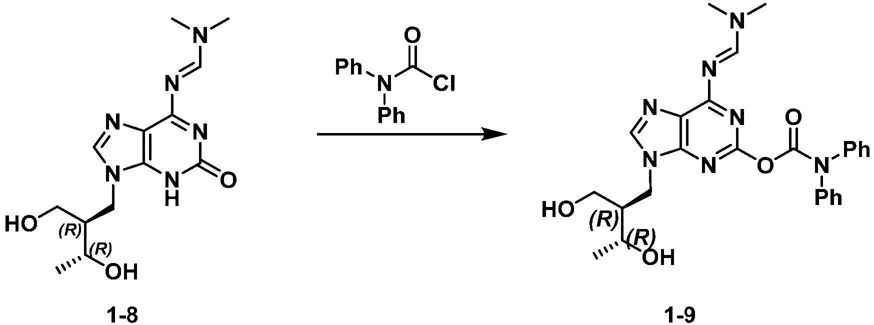

Compound 1-8 (24.6 mmol, 7.6 g) was placed in a clean, dry reaction flask. 260 mL of pyridine and N,N-diisopropylethylamine (1.5 equiv, 37.1 mmol, 4.8 g) were added. Under an ice bath, diphenylcarbamoyl chloride (1.5 equiv, 37.1 mmol, 8.57 g) was added, and the mixture was stirred at room temperature for 1 h. After the reaction, the reaction was quenched by dropwise addition of 100 mL of saturated sodium bicarbonate solution, extracted with dichloromethane, dried over anhydrous sodium sulfate, and concentrated. The resulting crude product was purified by silica gel column chromatography (gradient elution: dichloromethane/methanol = 100/0 - 90/10) to obtain off-white solid compound 1-9 (8.6 g, 17.1 mmol, 69% yield). Molecular formula of compound 1-9: *C*₂₆*H*₂₉*N*₇O₄, MW: 503.23, LC-MS measured: 504.3 (M+H).

### 5.9 Preparation of Intermediate 1-10

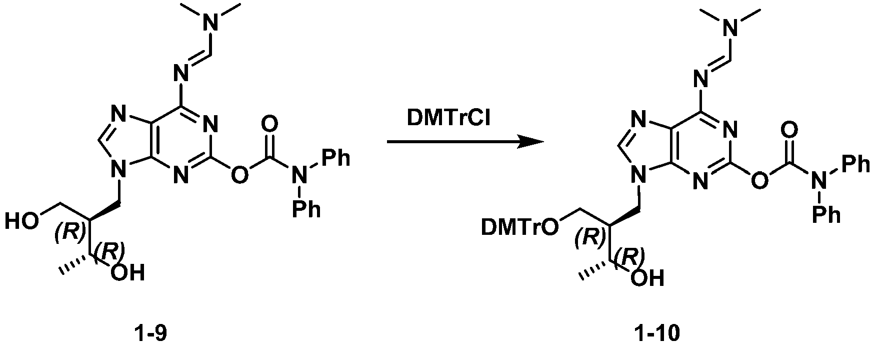

1-9 (17.1 mmol, 8.6 g) was placed in a clean, dry reaction flask. 86 mL of pyridine was added, and at room temperature, 4,4'-dimethoxytrityl chloride (1.3 equiv, 22.2 mmol, 7.5 g) was added. The mixture was then stirred at room temperature for 1 hour. After the reaction, most of the solvent was removed by concentration. 100 mL of ethyl acetate was added, and the mixture was washed with 100 mL of saturated sodium bicarbonate solution and 100 mL of saturated brine. The organic phase was dried, filtered, and concentrated. The resulting crude product was purified by silica gel column chromatography (gradient elution: dichloromethane/methanol = 100/0 - 90/10) to obtain pale yellow solid 1-10 (7.1 g, 8.8 mmol, 52% yield). Molecular formula of compound 1-10: *C*₄₇*H*₄₇*N*₇*O*₆, MW: 805.36, LC-MS measured: 806.5 (M+H).

### 5.10 Preparation of Compound SA000068 (Ggs)

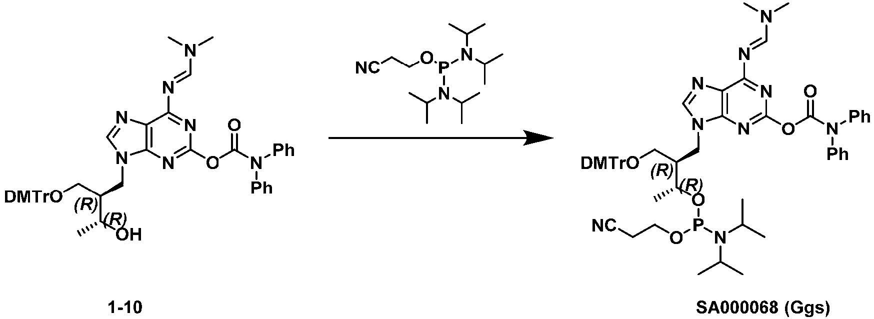

1-10 (1.24 mmol, 1.0 g) was placed in a clean, dry reaction flask, and 10 mL of anhydrous dichloromethane was added. At room temperature under an argon atmosphere, 2-cyanoethyl N,N,N',N'-tetraisopropylphosphorodiamidite (2.0 equiv, 2.48 mmol, 0.75 g) and 4,5-dicyanoimidazole (1.5 equiv, 1.86 mmol, 0.22 g) were added, and the mixture was stirred at room temperature for one hour. After the reaction, 10 mL of dichloromethane was added to the reaction mixture, and it was washed with 20 mL of saturated sodium bicarbonate solution. The organic phase was dried, filtered, and concentrated. The resulting crude product was purified by C18 reverse-phase column chromatography to obtain white solid SA000068 (0.8 g, 0.8 mmol, 64% yield). Molecular formula of compound SA000068: *C*₅₆*H*₆₄*N*₉*O*₇*P*, MW: 1005.47, LC-MS measured: 1006.7 (M+H). ¹H NMR (400 MHz, DMSO) δ 8.89 (s, 1H), 8.09 (s, 1H), 7.39 (dd, *J =* 8.8, 4.5 Hz, 8H), 7.32 - 7.21 (m, 4H), 7.21 - 7.11 (m, 3H), 7.08 (ddd, *J* = 8.6, 3.8, 2.2 Hz, 4H), 6.78 - 6.70 (m, 4H), 4.45 - 3.98 (m, 4H), 3.68 - 3.43 (m, 4H), 3.21 (s, 3H), 3.14 (s, 3H), 2.91 (ddd, *J =* 21.9, 9.6, 5.5 Hz, 1H), 2.70 (t, *J =* 5.9 Hz, 1H), 2.59 (ddd, *J =* 11.4, 8.6, 4.4 Hz, 2H), 1.36 - 1.27 (m, 1H), 1.20 (dd, *J =* 14.0, 6.4 Hz, 3H), 1.10 (d, *J =* 6.8 Hz, 6H), 1.02 (d, *J =* 6.7 Hz, 3H), 0.95 (d, *J =* 6.7 Hz, 3H). ³¹P NMR (162 MHz, DMSO) δ 147.06, 146.09.

### Example 6: Preparation of siRNA Conjugate

Using the solid-phase phosphoramidite method, the specially modified compounds prepared in the above steps and commercially purchased conventionally modified monomers (purchased from Shanghai Zhaowei Technology Development Co., Ltd.) were used to connect nucleoside monomers one by one in the 3'-5' direction according to the nucleotide sequence. The specially modified anti-off-target compound was placed in the seed region of the antisense strand (at any position from the 4th to the 8th from the 5' end). The connection of each nucleoside monomer included four reaction steps: deprotection, coupling, capping, and oxidation or sulfurization. The synthesis conditions used for the sense and antisense strands.

Instrument model: MerMade 12 Oligonucleotide synthesizer, Beijing Haijing 1 ml synthesis column, Cytiva SourceTM 15Q 4.6/100PE purification column.

Reagents used for synthesizing the siRNA conjugate were purchased from Suzhou Cole-Parmer.

A brief description of the synthesis is as follows:
The single-strand synthesis reaction proceeds in the 3'-5' direction and is completed on a solid-phase synthesizer. It includes four main reaction steps:
a. DMT deprotection reaction: Dichloroacetic acid is used to remove the protecting group DMT from the nucleotide to obtain a 5'-hydroxyl end;
b. Coupling reaction: The protected nucleoside phosphoramidite monomer is mixed with the activator 5-(ethylthio)-1H-tetrazole. The phosphoramidite group is activated. The 5'-hydroxyl, still protected by DMT, undergoes a condensation reaction with the 5'-hydroxyl linked to the solid support, forming a phosphite triester;
c. Oxidation reaction: Under the action of the oxidizing agent iodine, the phosphite triester obtained from the previous condensation reaction is converted into a more stable phosphate ester (i.e., oxidizing trivalent phosphorus to pentavalent phosphorus);
d. Thiolation reaction: Under the action of the sulfurizing agent PADS, the phosphite triester obtained from the previous condensation reaction is converted into a phosphorothioate ester (oxidation or thiolation is selected according to the sequence design).
e. Capping reaction: A very small number of 5'-hydroxyl groups may not have reacted during the condensation reaction (less than 2%). They are reacted with acetic anhydride and 1-methylimidazole to form an acetate ester cap that cannot participate in subsequent reactions, preventing further reaction. These short fragments can be removed during purification.

The above four steps are repeated in a cycle until the desired sequence is synthesized.

The main chemical reaction equations are as follows:
After the last nucleoside monomer is connected, the nucleic acid sequence linked to the solid support is sequentially subjected to cleavage, deprotection, purification, and desalting, followed by lyophilization to obtain the sense and antisense strands, wherein:
Cleavage and deprotection conditions are as follows: First, prepare the ammonolysis solution (a mixture of ammonia water:ethanol=3:1 to a volume of 2 mL). The solid support is added to the above reaction flask and shaken thoroughly to mix well.

Ammonolysis is carried out at 50°C in a constant temperature water bath for 16 hours.

After 16 hours of ammonolysis, the water bath is cooled to room temperature (25°C±2°C), filtered through a sintered glass funnel, the filtrate is collected in a roundbottom flask, and the filter cake is washed with 50% aqueous ethanol solution. The filtrates are collected, concentrated on a rotary evaporator, then transferred to a glass vial.

A small sample of the crude product is sent to the analysis department for crude LC-MS analysis. The detection method is as follows: A Waters Acquity UPLC-LTQ LCMS (column: ACQUITY UPLC BEH C18) is used to detect the purity of the above sense and antisense strands and analyze their molecular weights. The measured values are consistent with the theoretical values.

Purification and desalting conditions are as follows: An ion-exchange chromatography column is used for purification, in conjunction with a Cytiva HiPrepTM 26/10 desalting gel column for desalting, followed by lyophilization of the single strands. After lyophilization of the single strands, a sample needs to be taken for LC-MS measurement.

Finally, the obtained sense and antisense strands need to be annealed into a double strand.

Annealing operation is as follows: The purified sense and antisense strands are dissolved separately in water for injection to prepare solutions of 0.1 mg/mL - 40 mg/mL. They are mixed in equimolar ratios calibrated with a Thermo Nanodrop Eight, heated at 90°C for 5 minutes, and then slowly cooled down naturally to allow them to form a double-stranded structure through hydrogen bonds. A sample is taken to test the SEC purity of the product. The double-stranded sample is lyophilized.

### Example 7: Preparation of siRNA with Conjugated Delivery Moiety

### I. Preparation of GalNAc Targeting Moiety

### 1. Synthesis of Compound 2

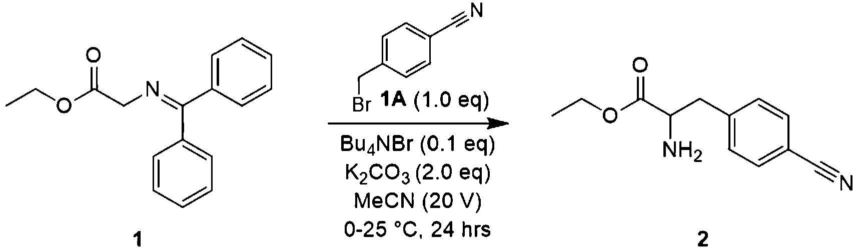

Compound 1 (30 g, 0.112 mol, CAS: 69555-14-2, commercially available from TCI), potassium carbonate (31 g, 0.224 mol), and tetrabutylammonium bromide (3.62 g, 0.011 mol) were added to 600 mL of acetonitrile. After mixing well, the mixture was cooled to 0°C, and compound 1A (22 g, 0.112 mol, CAS: 17201-43-3, commercially available from TCI) was gradually added. The mixture was then gradually warmed to 25°C and stirred for 24 hours. Monitored by thin-layer chromatography, after compound 1 was consumed, the mixture was filtered and the solvent was removed by rotary evaporation. The remaining solid was washed once with 300 mL of 2 M hydrochloric acid and 300 mL of tetrahydrofuran, respectively, and extracted three times with 300 mL of ethyl acetate. After extraction, the aqueous phase was adjusted to pH 8.0 with sodium carbonate, and extracted again three times with 300 mL of ethyl acetate. The organic phase was washed once with 300 mL of water and separated using a separatory funnel. The ethyl acetate phase was dried over anhydrous sodium sulfate and concentrated by rotary evaporation to obtain yellow solid compound 2 (14.1 g, 57.6% yield).

¹H NMR data for compound 2: 400 MHz, *CDCl*₃ 7.60 (d, *J* = 8.0 Hz, 2H), 7.34 (d, *J* = 8.0 Hz, 2H), 4.16 (q, *J =* 7.2 Hz, 2H), 3.41-3.34 (m, 1H), 3.16-2.88 (m, 2H), 1.24 (t, *J =* 7.2 Hz, 3H).

Mass spectral identification of compound 2 (*C*₁₂*H*₁₄*N*₂*O*₂, MW 218.1, [M+H] = 219.1).

### 2. Synthesis of Compound 3

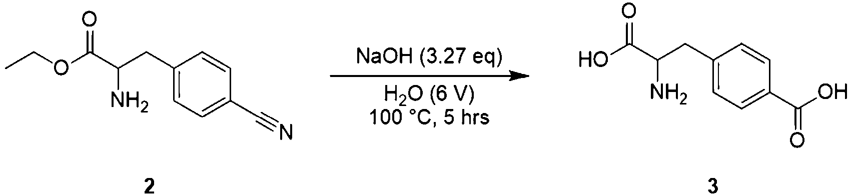

Compound 2 was added to 85 mL of water containing 8.46 g (0.212 mol) of sodium hydroxide. After replacing the oxygen in the system with nitrogen, the dissolved solution was heated to 100°C and stirred for 4 hours. After compound 2 had completely reacted, the reaction system was cooled to 50°C, the pH was adjusted to 7.0 with 12 M hydrochloric acid, and it was further cooled to 25°C. After stirring for one hour, the solvent was removed by rotary evaporation at 50°C to obtain yellow solid 3 (12.2 g, yield 90.3%).

Molecular formula of compound 3: *C*₁₀*H*₁₁*NO*₄, MW 209.1, [M+H] = 210.1.

### 3. Synthesis of Compound 3A

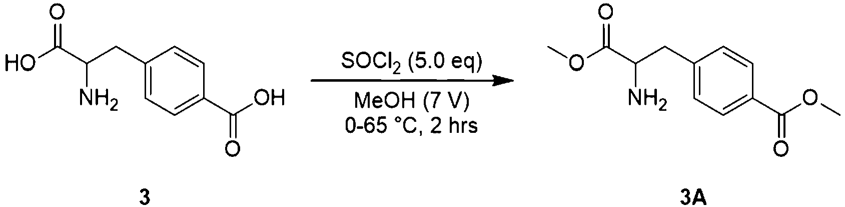

Synthesis method of compound 3A: 16.3 g (0.078 mol) of compound 3 was dissolved in 114 mL of methanol. At 0-5°C, 46.3 g of thionyl chloride (0.39 mol) was added. After stirring at 0-5°C for 30 minutes, the temperature was raised to 65°C and refluxed for 2 hours. After LCMS confirmed that compound 3 was consumed, the reaction system was concentrated and the pH was adjusted to 1-2 with concentrated hydrochloric acid. The mixture was extracted 3 times with 20 mL of dichloromethane; the pH of the aqueous phase was adjusted to pH 11-12 with solid potassium carbonate, and then extracted 3 times with 20 mL of dichloromethane. The organic phases were combined and evaporated to dryness. The resulting solid was purified by silica gel column chromatography (petroleum ether/ethyl acetate 50:1 to 1:1) to obtain 8.1 g of yellow oily compound 3A (on TLC, in petroleum ether:ethyl acetate=1:1 system, Rf value 0.3, reaction yield 43.8%).

¹H NMR data for compound 3A: 400 MHz, *CDCl*₃ 7.98 (d, *J =* 8.4 Hz, 2H), 7.28 (d, *J =* 8.4 Hz, 2H), 3.91 (s, 3H), 3.79-3.74 (m, 1H), 3.71 (s, 3H), 3.13 (dd, *J* = 14.4, 5.6 Hz, 1H), 2.92 (dd, *J* = 13.6, 8.0 Hz, 1H).

Mass spectral data for compound 3A: Molecular formula *C*₁₂*H*₁₅*NO*₄, MW 237.1, [M+H] = 238.1.

### 4. Synthesis of Compound 4

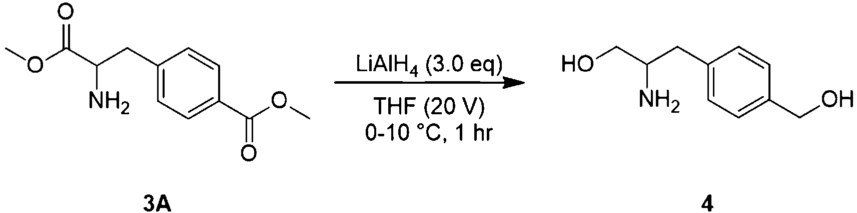

6.7 g of compound 3A (0.028 mol) was dissolved in 34 mL of tetrahydrofuran, and then added dropwise to 100 mL of tetrahydrofuran containing 3.22 g of lithium aluminum hydride (0.085 mol) (temperature 0-5°C), and stirred at 0-10°C for 1 hour. LCMS monitoring confirmed that compound 3A was consumed and a new compound was produced. After dropwise addition of 3 mL to quench the reaction (temperature 5-15°C), 9 mL of 15% aqueous sodium hydroxide solution, 9 mL of water, and 9 g of sodium sulfate were added sequentially, and the mixture was stirred for 30 minutes. It was then filtered through celite and concentrated to obtain yellow oily compound 4 (3.8 g, yield 74.2%).

¹H NMR data for compound 4: 400 MHz, DMSO-*d*₆ 7.32-7.02 (m, 4H), 5.10 (s, 1H), 4.55 (s, 1H), 4.45 (s, 2H), 3.30-3.23 (m, 1H), 3.20-3.11 (m, 1H), 2.89-2.78 (m, 1H), 2.64 (dd, *J =* 13.2, 5.6 Hz, 1H), 2.39 (dd, *J =* 13.2, 7.6 Hz, 1H).

Molecular formula of compound 4: *C*₁₀*H*₁₅*NO*₂ (MW 181.1, [M+H] = 182.2).

### 5. Synthesis of Compound 5

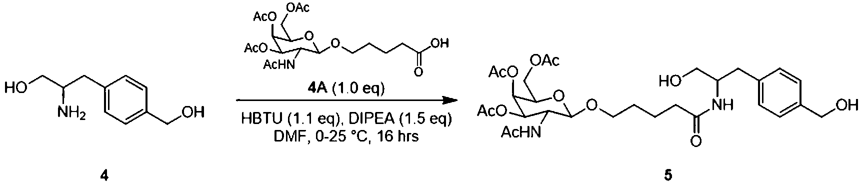

9.38 g of compound 4A (0.021 mol) was dissolved in 57 mL of N,N-dimethylformamide. 4.07 g (0.0315 mol) of DIPEA and 8.74 g of HBTU (0.023 mol) were added. Subsequently, at 0-10°C, 3.8 g of compound 4 (0.021 mol) was added, and the mixture was stirred at 0-10°C for 16 hours. After filtering the reaction system, the filtrate was purified by preparative high-performance liquid chromatography (Xtimate C18 preparative column, 250mm X 80mm, mobile phase water:acetonitrile = 10%-40%, 21 minutes) to obtain white solid 5 (5.6 g, yield 43.7%).

¹H NMR data for compound 5: 400 MHz, DMSO-*d*₆ 7.82 (d, *J* = 9.2 Hz, 1H), 7.60 (d, *J =* 8.4 Hz, 1H), 7.24-7.09 (m, 4H), 5.21 (d, *J* = 3.2 Hz, 1H), 5.10 (t, *J =* 5.6 Hz, 1H), 4.96 (dt, *J =* 11.2, 3.6 Hz, 1H), 4.79-4.71 (m, 1H), 4.52-4.39 (m, 3H), 4.08-3.96 (m, 3H), 3.93-3.80 (m, 2H), 3.71-3.61 (m, 1H), 3.28-3.20 (m, 1H), 2.87-2.76 (m, 1H), 2.61-2.54 (m, 1H), 2.10 (s, 3H), 2.03-1.96 (m, 5H), 1.89 (s, 3H), 1.76 (s, 3H), 1.52-1.27 (m, 4H).

Molecular formula of compound 5: *C*₂₉*H*₄₂*N*₂*O*₁₂, MW 610.2, [M+H] = 611.2.

### 6. Synthesis of Compound 6

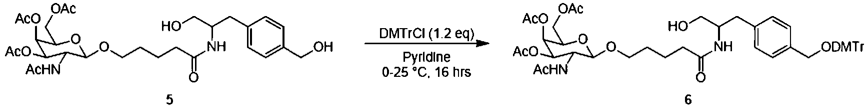

5.6 g of compound 5 (9.17 mmol) was added to 50 mL of pyridine. Subsequently, at 0-5°C, 3.73 g of 4,4'-dimethoxytrityl chloride (11.0 mmol) was added. After warming to 25°C, the reaction was stirred for 16 hours. The reaction system was filtered, and the filtrate was evaporated to dryness and separated by preparative high-performance liquid chromatography (Waters Xbridge BEH C18 column, 250mm X 50 mm, 10µm), mobile phase water:acetonitrile 45% to 65%, 10 minutes). After purification, the sample was freeze-dried to obtain 1.2 g of white solid 6 (yield 14.3%).

¹H NMR data for compound 6: 400 MHz, DMSO-*d*₆ 7.77 (dd, *J =* 9.2, 3.2 Hz, 1H), 7.58 (d, *J* = 8.4 Hz, 1H), 7.42-7.36 (m, 2H), 7.33-7.24 (m, 6H), 7.23-7.17 (m, 3H), 7.17-7.12 (m, 2H), 6.91-6.84 (m, 4H), 5.21-5.12 (m, 1H), 4.97-4.88 (m, 1H), 4.78-4.69 (m, 1H), 4.43 (dd, *J =* 8.4, 7.2 Hz, 1H), 4.03-3.91 (m, 5H), 3.91-3.77 (m, 2H), 3.70 (s, 6H), 3.67-3.57 (m, 1H), 3.38-3.32 (m, 1H), 3.27-3.19 (m, 1H), 2.84-2.75 (m, 1H), 2.60-2.51 (m, 1H), 2.08-2.03 (m, 3H), 2.01-1.90 (m, 5H), 1.85 (s, 3H), 1.76-1.67 (m, 3H), 1.47-1.24 (m, 4H).

Compound 6 is unstable in aqueous solution and prone to degradation, so complete mass spectral data for compound 6 could not be obtained.

### 7. Synthesis of Compound SA000004

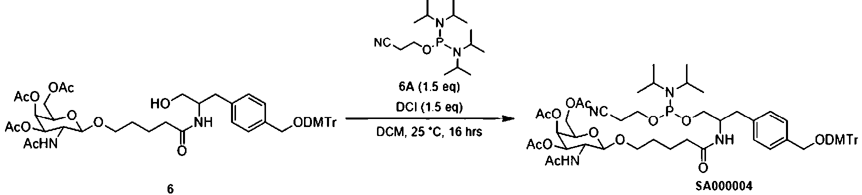

1 g of compound 6 (1.09 mmol) and 1H-imidazole-4,5-dicarbonitrile (194 mg, 1.64 mmol) were added to 1000 mL of dichloromethane. At 25°C, 496 mg of compound 6A (1.64 mmol) was added, and the mixture was stirred at 25°C for 16 hours. After TLC confirmed that compound 6 was consumed, a new compound was observed. The reaction system was evaporated to dryness, and separation by silica gel column chromatography (petroleum ether:ethyl acetate, 20:1 to 1:1) yielded 211 mg of white compound SA000004 (yield 17.3%).

¹H NMR data for compound SA000004: 400 MHz, *CD*₃*CN* 7.52-7.45 (m, 2H), 7.40-7.29 (m, 6H), 7.29-7.19 (m, 5H), 6.94-6.83 (m, 4H), 6.53-6.37 (m, 2H), 5.27 (d, *J =* 3.2 Hz, 1H), 5.04-4.93 (m, 1H), 4.47 (dd, *J =* 15.6, 8.4 Hz, 1H), 4.26-4.14 (m, 1H), 4.12-4.01 (m, 4H), 4.01-3.69 (m, 11H), 3.68-3.34 (m, 5H), 2.96-2.85 (m, 1H), 2.79-2.69 (m, 1H), 2.66 (t, *J* = 5.6 Hz, 2H), 2.11-2.04 (m, 5H), 1.98-1.95 (m, 3H), 1.91 (s, 3H), 1.84-1.79 (m, 3H), 1.63-1.32 (m, 4H), 1.22-1.09 (m, 12H).

### II. Preparation of siRNA Conjugate

Using the solid-phase phosphoramidite method, the specially modified compounds prepared in the above steps and commercially purchased conventionally modified monomers (purchased from Shanghai Hongene Biotech Corporation.) were used to connect nucleoside monomers one by one in the 3'-5' direction according to the nucleotide sequence. The specially modified anti-off-target compound was placed in the seed region of the antisense strand (at any position from the 4th to the 8th from the 5' end), and the delivery monomer compound was freely placed at the 3' end or 5' end like a normal monomer. The connection of each nucleoside monomer included four reaction steps: deprotection, coupling, capping, and oxidation or sulfurization. The synthesis conditions used for the sense and antisense strands.

Instrument model: MerMade 12 Oligonucleotide synthesizer, Beijing Haijing 1 ml synthesis column, Cytiva SourceTM 15Q 4.6/100PE purification column.

Reagents used for synthesizing the siRNA conjugate were purchased from Suzhou Cole-Parmer.

A brief description of the synthesis is as follows:
The single-strand synthesis reaction proceeds in the 3'-5' direction and is completed on a solid-phase synthesizer. It includes four main reaction steps:
a. DMT deprotection reaction: Dichloroacetic acid is used to remove the protecting group DMT from the nucleotide to obtain a 5'-hydroxyl end;
b. Coupling reaction: The protected nucleoside phosphoramidite monomer is mixed with the activator 5-(ethylthio)-1H-tetrazole. The phosphoramidite group is activated. The 5'-hydroxyl, still protected by DMT, undergoes a condensation reaction with the 5'-hydroxyl linked to the solid support, forming a phosphite triester;
c. Oxidation reaction: Under the action of the oxidizing agent iodine, the phosphite triester obtained from the previous condensation reaction is converted into a more stable phosphate ester (i.e., oxidizing trivalent phosphorus to pentavalent phosphorus);
d. Thiolation reaction: Under the action of the sulfurizing agent PADS, the phosphite triester obtained from the previous condensation reaction is converted into a phosphorothioate ester (oxidation or thiolation is selected according to the sequence design).
e. Capping reaction: A very small number of 5'-hydroxyl groups may not have reacted during the condensation reaction (less than 2%). They are reacted with acetic anhydride and 1-methylimidazole to form an acetate ester cap that cannot participate in subsequent reactions, preventing further reaction. These short fragments can be removed during purification.

The above four steps are repeated in a cycle until the desired sequence is synthesized.

The main chemical reaction equations are as follows:
After the last nucleoside monomer is connected, the nucleic acid sequence linked to the solid support is sequentially subjected to cleavage, deprotection, purification, and desalting, followed by lyophilization to obtain the sense and antisense strands, wherein:
Cleavage and deprotection conditions are as follows: First, prepare the ammonolysis solution (a mixture of ammonia water:ethanol=3:1 to a volume of 2 mL). The solid support is added to the above reaction flask and shaken thoroughly to mix well.

Ammonolysis is carried out at 50°C in a constant temperature water bath for 16 hours.

After 16 hours of ammonolysis, the water bath is cooled to room temperature (25°C±2°C), filtered through a sintered glass funnel, the filtrate is collected in a roundbottom flask, and the filter cake is washed with 50% aqueous ethanol solution. The filtrates are collected, concentrated on a rotary evaporator, then transferred to a glass vial.

A small sample of the crude product is sent to the analysis department for crude LC-MS analysis. The detection method is as follows: A Waters Acquity UPLC-LTQ LCMS (column: ACQUITY UPLC BEH C18) is used to detect the purity of the above sense and antisense strands and analyze their molecular weights. The measured values are consistent with the theoretical values.

Purification and desalting conditions are as follows: An ion-exchange chromatography column is used for purification, in conjunction with a Cytiva HiPrepTM 26/10 desalting gel column for desalting, followed by lyophilization of the single strands. After lyophilization of the single strands, a sample needs to be taken for LC-MS measurement.

Finally, the obtained sense and antisense strands need to be annealed into a double strand.

Annealing operation is as follows: The purified sense and antisense strands are dissolved separately in water for injection to prepare solutions of 0.1 mg/mL - 40 mg/mL. They are mixed in equimolar ratios calibrated with a Thermo Nanodrop Eight, heated at 90°C for 5 minutes, and then slowly cooled down naturally to allow them to form a double-stranded structure through hydrogen bonds. A sample is taken to test the SEC purity of the product. The double-stranded sample is lyophilized.

### Example 8: In Vitro Evaluation of On-target and Anti-Off-target Activity of Safe01

In this example, the effect of having the chemical modification represented by (II) at the nucleotide position of the 6th or 7th position of the 5' region of different antisense strands on the on-target and anti-off-target activity of siRNA was verified.

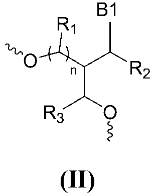

In formula (II), B1 can be A, G, C, or U.

When B1 is the base A, the chemically modified base A is called Ago; when B1 is the base G, the chemically modified base G is called Ggo; when B1 is the base C, the chemically modified base C is called Cgo; when B1 is the base U, the chemically modified base U is called Ugo.

It can be seen that different structures are formed when B1 is different. In this example, the on-target and anti-off-target activities of siRNA containing Ggo, Ago, Ugo, Cgo, or Ggs were verified. For convenience of description in this example, the general term Safe01 was used, which includes Ggo, Ago, Ugo, or Cgo in Table 1 or Table 2, whose specific structures have been given above.

In this example, a GNA chemical modification introduced into base A forms Agn, a GNA chemical modification introduced into base G forms Ggn, a GNA chemical modification introduced into base U forms Ugn, and a GNA chemical modification introduced into base C forms Cgn.

In this example, the unmodified sequences used for the modifications in Table 1 below are as follows in the 5'-3' direction. For details on the sequence modifications, see Table 1 below.
siRNA-1: Sense strand: UGACAAGAAUCCUCACAAU (SEQ ID NO: 1), Antisense strand: AUUGUGAGGAUUCUUGUCAAC (SEQ ID NO: 2);
siRNA-2: Sense strand: CCUUGAGGCAUACUUCAAA (SEQ ID NO: 3), Antisense strand: UUUGAAGUAUGCCUCAAGGUU (SEQ ID NO: 4);
siRNA-3: Sense strand: GUGUGCACUUCGCUUCACA (SEQ ID NO: 5), Antisense strand: UGUGAAGCGAAGUGCACACUU (SEQ ID NO: 6);
siRNA-4: Sense strand: ACUGAAGCAUUUGAUGCAA (SEQ ID NO: 7), Antisense strand: UUGCAUCAAAUGCUUCAGUGU (SEQ ID NO: 8);
siRNA-5: Sense strand: AUAACUCACUAUAAUUACU (SEQ ID NO: 9), Antisense strand: AGUAAUUAUAGUGAGUUAUUU (SEQ ID NO: 10).

A dual-luciferase reporter gene assay was used to detect the on-target and anti-off-target activities of the compounds in different sequences. HEK293 cells were cultured in DMEM high-glucose medium containing 10% fetal bovine serum at 37°C with 5% *CO*₂. Following the product manual, reporter gene plasmids and siRNAs at different concentrations were co-transfected into cells using Lipofectamine 2000 (ThermoFisher, 11668019). The siRNA sequences are shown in Table 1. After 24 hours of transfection, detection was performed using a dual-luciferase assay kit (Yeasen, 11405ES80). A sequence complementary to the antisense strand (for the on-target reporter plasmid) and five tandem repeats of a sequence identical to positions 12-19 of the 5' end of the sense strand (for the off-target reporter plasmid) were inserted into the 3' untranslated region of the Renilla luciferase gene in the reporter plasmids, respectively. The reporter plasmids also contained firefly luciferase as an internal control. The group transfected with only the plasmid served as the control group. The results are shown in Table 2. The results show that siRNAs containing Safe01 retained on-target activity in vitro and significantly reduced off-target activity; results from tests on different targets indicate that the on-target and anti-off-target activities of Safe01 are superior to GNA.

**Table 1: In Vitro Reporter Assay Sequences**

| Com pou nd No. | Sense Strand No. | Modified Sense Strand Sequence 5'-3' | Antisens e Strand No. | Modified Antisense Strand Sequence 5'-3' |
|---|---|---|---|---|
| SD0 032 35 | SP003 212 | UmsGmsAm CmAfAm GfAfAf UmCmCm UmCmAm CmAmAm Um | SG00321 8 | AmsUfsUm GmUmGf AmGmGm AmUmUm CmUfUm GfUmCm AmsAmsCm |
| SD0 037 25 | SP003 212 | UmsGmsAm CmAfAm GfAfAf UmCmCm UmCmAm CmAmAm Um | SG00352 5 | AmsUfsUm GmUm(Ggo)AmGmGm AmUmUm CmUfUm GfUmCm AmsAmsCm |
| SD0 037 26 | SP003 212 | UmsGmsAm CmAfAm GfAfAf UmCmCm UmCmAm CmAmAm Um | SG00352 6 | AmsUfsUm GmUm(Ggn)AmGmGm AmUmUm CmUfUm GfUmCm AmsAmsCm |
| SD0 032 36 | SP003 212 | UmsGmsAm CmAfAm GfAfAf UmCmCm UmCmAm CmAmAm Um | SG00321 9 | AmsUfsUm GmUmGf (Ago)GmGm AmUmUm CmUfUm GfUmCm AmsAmsCm |
| SD0 032 52 | SP003 212 | UmsGmsAm CmAfAm GfAfAf UmCmCm UmCmAm CmAmAm Um | SG00325 3 | AmsUfsUm GmUmGf (Agn) GmGm AmUmUm CmUfUm GfUmCm AmsAmsCm |
| SD0 032 38 | SP003 213 | CmsCmsUm UmGmAm GfGfCf AmUmAm CmUmUm CmAmAm Am | SG00322 1 | UmsUfsUm GmAmAf GmUmAm UmGmCm CmUfCm AfAmGm GmsUmsUm |
| SD0 032 39 | SP003 213 | CmsCmsUm UmGmAm GfGfCf AmUmAm CmUmUm CmAmAm Am | SG00322 2 | UmsUfsUm GmAm(Ago) GmUmAm UmGmCm CmUfCm AfAmGmGmsUmsUm |
| SD0 032 53 | SP003 213 | CmsCmsUm UmGmAm GfGfCf AmUmAm CmUmUm CmAmAm Am | SG00325 4 | UmsUfsUm GmAm(Agn) GmUmAm UmGmCm CmUfCm AfAmGm GmsUmsUm |
| SD0 037 27 | SP003 213 | CmsCmsUm UmGmAm GfGfCf AmUmAm CmUmUm CmAmAm Am | SG00352 7 | UmsUfsUm GmAmAf (Ggo)UmAm UmGmCm CmUfCm AfAmGm GmsUmsUm |
| SD0 051 18 | SP003 213 | CmsCmsUm UmGmAm GfGfCf AmUmAm CmUmUm CmAmAm Am | SG00458 6 | UmsUfsUm GmAmAf (Ggs)UmAm UmGmCm CmUfCm AfAmGm GmsUmsUm |
| SD0 037 28 | SP003 213 | CmsCmsUm UmGmAm GfGfCf AmUmAm CmUmUm CmAmAm Am | SG00352 8 | UmsUfsUm GmAmAf (Ggn)UmAm UmGmCm CmUfCm AfAmGm GmsUmsUm |
| SD0 032 41 | SP003 214 | GmsUmsGm UmGmCm AfCfUf UmCmGm CmUmUm CmAmCm Am | SG00221 2 | UmsGfsUm GmAmAf GmCfGf AmAmGm UmGfCm AfCmAm CmsUmsUm |
| SD0 032 42 | SP003 214 | GmsUmsGm UmGmCm AfCfUf UmCmGm CmUmUm CmAmCm Am | SG00251 2 | UmsGfsUm GmAm(Agn) GmCfGf AmAmGm UmGfCm AfCmAm CmsUmsUm |
| SD0 032 43 | SP003 214 | GmsUmsGm UmGmCm AfCfUf UmCmGm CmUmUm CmAmCm Am | SG00322 4 | UmsGfsUm GmAm(Ago) GmCmGm AmAmGm UmGfCm AfCmAm CmsUmsUm |
| SD0 037 29 | SP003 214 | GmsUmsGm UmGmCm AfCfUf UmCmGm CmUmUm CmAmCm Am | SG00352 9 | UmsGfsUm GmAmAf (Ggo)CfGf AmAmGm UmGfCm AfCmAm CmsUmsUm |
| SD0 051 16 | SP003 214 | GmsUmsGm UmGmCm AfCfUf UmCmGm CmUmUm CmAmCm Am | SG00458 4 | UmsGfsUm GmAmAf (Ggs)CfGf AmAmGm UmGfCm AfCmAm CmsUmsUm |
| SD0 037 30 | SP003 214 | GmsUmsGm UmGmCm AfCfUf UmCmGm CmUmUm CmAmCm Am | SG00353 0 | UmsGfsUm GmAmAf (Ggn)CfGf AmAmGm UmGfCm AfCmAm CmsUmsUm |
| SA0 024 3 | SP002 236 | AmsCmsUmGmAmAm GfCfAfUmUmUmGmA mUmGmCmAmAm | SG00224 5 | UmsUfsGmCmAmUfCmAmA mAmUmGmCmUfUmCfAmG mUmsGmsUm |
| SD0 037 31 | SP002 236 | AmsCmsUmGmAmAm GfCfAfUmUmUmGmA mUmGmCmAmAm | SG00353 1 | UmsUfsGmCmAm(Ugo) CmAmAmAmUmGmCmUfU mCfAmGmUmsGmsUm |
| SD0 037 32 | SP002 236 | AmsCmsUmGmAmAm GfCfAfUmUmUmGmA mUmGmCmAmAm | SG00353 2 | UmsUfsGmCmAm(Ugn) CmAmAmAmUmGmCmUfU mCfAmGmUmsGmsUm |
| SD0 037 33 | SP002 236 | AmsCmsUmGmAmAm GfCfAfUmUmUmGmA mUmGmCmAmAm | SG00353 3 | UmsUfsGmCmAmUf(Cgo) AmAmAmUmGmCmUfUmCf AmGmUmsGmsUm |
| SD0 037 34 | SP002 236 | AmsCmsUmGmAmAm GfCfAfUmUmUmGmA mUmGmCmAmAm | SG00353 4 | UmsUfsGmCmAmUf(Cgn) AmAmAmUmGmCmUfUmCf AmGmUmsGmsUm |
| SA0 036 2 | SP002 355 | AmsUmAmAmCmUmC fAfCfUmAmUmAmAm UmUmAmCmUm | SG00236 4 | AmsGfsUmAmAmUfUmAm UmAmGmUmGmAfGmUfU mAmUmsUmsUm |
| SD0 037 35 | SP003 674 | AmsUmAmAmCmUmC fAfCfUmAmUmAmAm UmUmAmCmUm | SG00353 5 | AmsGfsUmAmAm(Ugo)UmA mUmAmGmUmGmAfGmUf UmAmUmsUmsUm |
| SD0 037 36 | SP003 674 | AmsUmAmAmCmUmC fAfCfUmAmUmAmAm UmUmAmCmUm | SG00353 6 | AmsGfsUmAmAm(Ugn)UmA mUmAmGmUmGmAfGmUf UmAmUmsUmsUm |
| SD0 037 37 | SP003 674 | AmsUmAmAmCmUmC fAfCfUmAmUmAmAm UmUmAmCmUm | SG00353 7 | AmsGfsUmAmAmUf(Ugo)A mUmAmGmUmGmAfGmUf UmAmUmsUmsUm |
| SD0 037 38 | SP003 674 | AmsUmAmAmCmUmC fAfCfUmAmUmAmAm UmUmAmCmUm | SG00353 8 | AmsGfsUmAmAmUf(Ugn)A mUmAmGmUmGmAfGmUf UmAmUmsUmsUm |

**Table 2: In Vitro Reporter Assay Results**

| Target | Compoun d No. | Annotation | GSCM *IC*₅₀(nM) | GSSM *IC*₅₀(nM) |
|---|---|---|---|---|
| Target 1 | SD00323 5 | Parent strand | 0.2474 | 0.3964 |
| | SD00372 5 | Safe01 at position 6 of antisense strand | 0.544 | >50 |
| | SD00372 6 | GNA at position 6 of antisense strand | 1.013 | >50 |
| | SD00323 6 | Safe01 at position 7 of antisense strand | 0.1748 | >50 |
| | SD00325 2 | GNA at position 7 of antisense strand | 0.2481 | >50 |
| Target 2 | SD00323 8 | Parent strand | 0.05785 | 0.7259 |
| | SD00372 7 | Safe01 at position 7 of antisense strand | 0.3259 | >50 |
| | SD00511 8 | Safe01 at position 7 of antisense strand (Ggs) | 0.042 | >50 |
| | SD00372 8 | GNA at position 7 of antisense strand | >50 | >50 |
| Target 3 | SD00324 1 | Parent strand | 0.405 | 0.289 |
| | SD00324 3 | Safe01 at position 6 of antisense strand | 0.602 | 0.656 |
| | SD00324 2 | GNA at position 6 of antisense strand | 1.569 | 3.057 |
| | SD00372 9 | Safe01 at position 7 of antisense strand | 2.133 | >50 |
| | SD00511 6 | Safe01 at position 7 of antisense strand (Ggs) | 0.563 | >50 |
| | SD00373 0 | GNA at position 7 of antisense strand | >50 | >50 |
| Target 4 | SA00243 | Parent strand | 0.02446 | 1.121 |
| | SD00373 1 | Safe01 at position 6 of antisense strand | 0.3983 | >50 |
| | SD00373 2 | GNA at position 6 of antisense strand | 0.4403 | >50 |
| | SD00373 3 | Safe01 at position 7 of antisense strand | 0.0776 | >50 |
| | SD00373 4 | GNA at position 7 of antisense strand | 0.9543 | >50 |
| Target 5 | SA00362 | Parent strand | 0.06057 | >50 |
| | SD00373 5 | Safe01 at position 6 of antisense strand | 0.3231 | >50 |
| | SD00373 6 | GNA at position 6 of antisense strand | 0.5817 | >50 |
| | SD00373 7 | Safe01 at position 7 of antisense strand | 0.8208 | >50 |
| | SD00373 8 | GNA at position 7 of antisense strand | >50 | >50 |

### Example 9: In Vivo Activity Test of Safe01-Modified Compounds Using C57BL/6-HBV (Hepatitis B Virus) Transgenic Mice

C57BL/6-HBV transgenic mice were selected from Beijing Vital River Laboratory Animal Technology Co., Ltd.: A linearized fragment of 1.28 times the length of HBV (Type A, GenBank: AF305422.1) was injected into the pronuclei of C57BL/6NCrl mouse embryos to obtain transgenic positive mice. Through peripheral blood HBV copy number analysis, founder mice with copy numbers reaching 10⁷-10⁸ eq/ml were retained, and an HBV transgenic mouse strain was established by mating hemizygous mice with wild-type C57BL/6NCrl mice.

The original siRNA sequence compound was SD003254, and the Safe01 anti-off-target modified compound was SD003257, with their sequences shown in Table 3.

C57BL/6-HBV transgenic mice were divided into three groups, with 5 mice per group. On day -3, submandibular blood was collected from the animals. After blood collection, it was centrifuged at 5000 rpm, 4°C for 10 minutes. The serum was diluted with PBS, with the dilution factor determined based on the actual situation. The diluted samples were then sent to Beijing Dian Medical Laboratory for HBV DNA detection. The measurement results were back-calculated according to the dilution factor. The quantitative detection method for HBV DNA was quantitative PCR with a fluorescent probe. Based on the HBV DNA levels, the animals were grouped. On day 0, the three groups of animals were subcutaneously injected with vehicle (phosphate-buffered saline), SD003254, and SD003257, respectively, at a dose of 3 mg/kg body weight, with a single injection. On day 56 of the experiment, blood was collected again to detect HBV DNA levels, to compare the activity of SD003254 and SD003257.

The level (%) of HBV DNA relative to the vehicle group before and after administration is shown in Figure 1.

On day 56 after treatment, compared to the vehicle group, the inhibition rates of HBV DNA in the SD003254 and SD003257 administration groups were approximately 92.8% and 87.6%, respectively, showing basically consistent activity (P value > 0.5, no statistical difference). This indicates that the Safe01 molecular modification has no significant effect on the long-term in vivo efficacy of the compound.

**Table 3: Compound Sequence Information**

| **Dupl ex No.** | **Sense Strand No.** | **Sense Strand Sequence 5'-3'** | **Antisense Strand No.** | **Antisense Strand Sequence 5'-3'** |
|---|---|---|---|---|
| SD0 0325 4 | SP0032 46 | FINs- GmsUmsGmUmGmCmA fCfUfUmCmGmCmUmU mCmAmCmsAms-FIN | SG003258 | VPUmsGfsUmGmAmAf GmCmGmAmAmGmUm GfCmAfCmAmCmsUms Um |
| SD0 0325 7 | SP0032 46 | FINs- GmsUmsGmUmGmCmA fCfUfUmCmGmCmUmU mCmAmCmsAms-FIN | SG003257 | VPUmsGfsUmGmAm(A go)GmCmGmAmAmGm UmGfCmAfCmAmCmsU msUm |

In this example, the conjugating group (FIN) conjugated to the siRNA is:

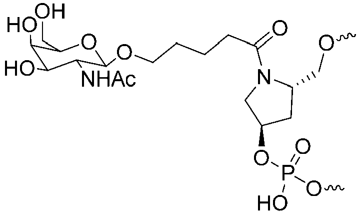

### Example 10: Analysis of Anti-Off-target Activity of Safe01-Modified Compounds Using RNA-seq

RNA-seq, or transcriptome sequencing technology, uses high-throughput sequencing for analysis, used to analyze differentially expressed genes. The standard workflow starts with RNA extraction from the lab, followed by mRNA enrichment or ribosomal RNA removal, cDNA reverse transcription, and preparation of sequencing libraries ligated with adapters. Next, this library is sequenced on a high-throughput sequencing platform, with each sample typically being sequenced to 10,000,000-30,000,000 reads. Finally, the data obtained from the experiment is analyzed by aligning or assembling the sequencing reads to the transcriptome, quantifying the reads covering the transcripts, filtering and normalizing between samples, and using statistical models to describe the expression level differences of each gene between sample groups.

Using an in vitro transfection method, SD003254 and SD003257 were transfected into HEK293 cells. RNA-seq was used to analyze the differences in gene expression levels in HEK293 cells to compare the improvement of off-target effects by the Safe01-modified compound. HEK293 cells were cultured in DMEM high-glucose medium containing 10% fetal bovine serum at 37°C with 5% *CO*₂. For transfection, HEK293 cells were seeded in 6-well plates at a density of 600,000 cells per well in 2 mL of medium. Following the product manual, siRNA was transfected using Lipofectamine RNAiMAX (ThermoFisher, 13778150) at a final concentration of 10 nM. Cells were collected 24 hours after treatment and sent to Suzhou Genewiz Biotechnology Co., Ltd. for RNA-seq analysis.

The RNA-seq results are shown in Table 4. The results show that the Safe01-modified compound SD003257 had fewer up-regulated and down-regulated genes compared to SD003254, indicating that Safe01 improved the off-target effect.

**Table 4: RNA-seq Analysis Results of Test Substances**

| Compoun d No. | Annotation | No. of Up-regulated Genes | No. of Down-regulated Genes |
|---|---|---|---|
| SD00325 4 | Parent | 25 | 24 |
| SD00325 7 | Safe01 at position 6 of antisense strand | 16 | 21 |

### Example 11: In Vitro Comparative Evaluation of On-target Activity of Safe01 and A0360

In this example, the effect on the on-target activity of siRNA was comparatively evaluated for chemical modifications of (II) and A0360 at the nucleotide position of the 6th position of the 5' region of the antisense strand.

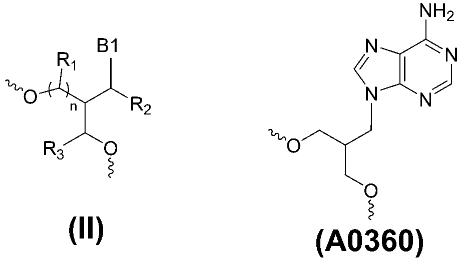

In formula (II), B1 can be A, G, C, or U.

Different structures are formed when B1 is different. For convenience of description in this example, the general term Safe01 was used, which includes Ago (when B is the base A, the chemically modified base A is called Ago) in Table 5 or Table 6, whose specific structure has been given above.

The on-target activity of the sequences was evaluated using the Dual-Glo^{®} Luciferase Assay System. On-target activity was assessed by co-transfecting 10 ng of the on-target reporter gene plasmid (with the sequence GSCM, which is perfectly complementary to the antisense strand, inserted into the 3' untranslated region of the Renilla luciferase gene in the reporter plasmid) and siRNA diluted in 2.5-fold serial dilutions from 20 nM to 0.00524 nM into cells using Lipofectamine 2000 (ThermoFisher, 11668019). HEK293 cells were cultured in DMEM high-glucose medium containing 10% fetal bovine serum at 37°C with 5% *CO*₂. The siRNA sequences are shown in Table 5. After 24 hours of transfection, detection was performed using a dual-luciferase assay kit (Promega, E1980). The reporter plasmids also contained firefly luciferase as an internal control. The group transfected with only the plasmid served as the control group.

The Renilla luciferase signal reading of each well was normalized by the ratio to the firefly luciferase (control) signal, and then the relative levels at different concentrations were calculated by comparing with cells transfected with the same plasmid but not treated with siRNA. The detection results are shown in Table 6. The results show that the siRNA containing Safe01 retained on-target activity in vitro that was basically the same as the original sequence, and the on-target activity of Safe01 was superior to A0360.

**Table 5: Compound Sequence Information**

| Dupl ex No. | Sense Strand No. | Sense Strand Sequence 5'-3' | Antisense Strand No. | Antisense Strand Sequence 5'-3' |
|---|---|---|---|---|
| SD00 3241 | SP0032 14 | GmsUmsGmUmGmC mAfCfUfUmCmGmC mUmUmCmAmCmA m | SG00221 2 | UmsGfsUmGmAmAfGmCfGf AmAmGmUmGfCmAfCmAm CmsUmsUm |
| SD00 3243 | SP0032 14 | GmsUmsGmUmGmC mAfCfUfUmCmGmC mUmUmCmAmCmA m | SG00322 4 | UmsGfsUmGmAm(Ago) GmCmGmAmAmGmUmGfC mAfCmAmCmsUmsUm |
| SD00 4083-1 | SP0032 14 | GmsUmsGm UmGmCm AfCfUf UmCmGm CmUmUm CmAmCm Am | SG00378 0-1 | UmsGfsUm GmAm(A0360) GmCmGm AmAmGm UmGfCm AfCmAm CmsUmsUm |

**Table 6: Relative Expression Level Analysis Results of Test Substances**

| | | | | | | |
|---|---|---|---|---|---|---|
| siRNA Concentration nM | 20 | 8 | 3.2 | 1.28 | 0.512 | |
| SD003241 | 15.71% | 12.52% | 13.44% | 29.60% | 33.51% | |
| SD003243 | 18.47% | 13.84% | 15.03% | 32.13% | 51.57% | |
| SD004083-1 | 19.59% | 16.46% | 22.91% | 47.70% | 75.77% | |
| siRNA Concentration nM | 0.2048 | 0.08192 | 0.03276 8 | 0.013107 2 | 0.00524 | 0 |
| SD003241 | 78.20% | 89.22% | 99.33% | 110.92% | 117.05 % | 100.00% |
| SD003243 | 95.13% | 105.84% | 111.19% | 110.64% | 117.19 % | 100.00% |
| SD004083-1 | 102.31% | 92.78% | 82.33% | 96.77% | 100.50 % | 100.00% |

The present disclosure has been described in detail above. The purpose is to enable those skilled in the art to understand the content of the present disclosure and to practice it, and it is not intended to limit the protection scope of the present disclosure. Any equivalent changes or modifications made in accordance with the spirit and substance of the present disclosure shall be covered by the protection scope of the present disclosure.

## Claims

1. A single-stranded or double-stranded oligonucleotide, wherein each strand has 15 to 35 nucleotides, and at least one nucleotide position of the oligonucleotide other than the termini comprises a chemical modification represented by formula (II) or a tautomer modification thereof, 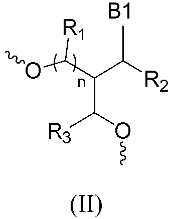
wherein, B1 is a natural nucleobase, a modified nucleobase, a universal base or an H atom;
*R*₁, *R*₂ and *R*₃ are each independently selected from H, OH, halogen, *NH*₂, *C*₁ - *C*₆ alkyl, *C*₁ - *C*₆ alkoxy, *C*₂ - *C*₆ alkenyl, *C*₂ - *C*₆ alkynyl, *S - CH*₃, *NCH*₃(*CH*₃), *OCH*₂*CH*₂*OCH*₃, and -O-alkylamino;
n is 1, 2 or 3;
wherein, the chemical modification represented by said formula (II) is not

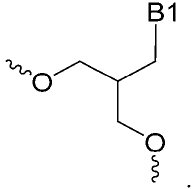

2. The single-stranded or double-stranded oligonucleotide according to claim 1, **characterized in that**: the oligonucleotide is an siRNA, which comprises a sense strand and an antisense strand, each strand having 15 to 35 nucleotides, and the antisense strand comprises a chemical modification represented by formula (II-1), (II-2), (II-3) or (II-4) or a tautomer modification thereof at at least one nucleotide position from position 2 to position 8 of its 5' region, 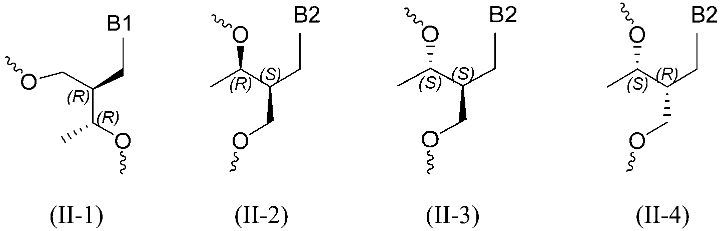 wherein: B1, B2 are each a natural nucleobase, a modified nucleobase, a universal base or an H atom.

3. The single-stranded or double-stranded oligonucleotide according to claim 2, **characterized in that**: the chemical modification is selected from any one of the following structures:

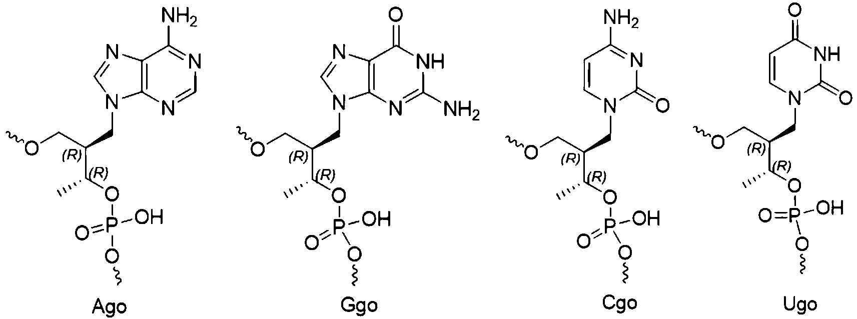

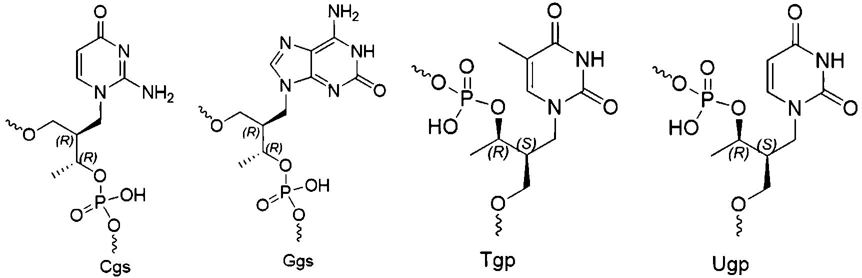

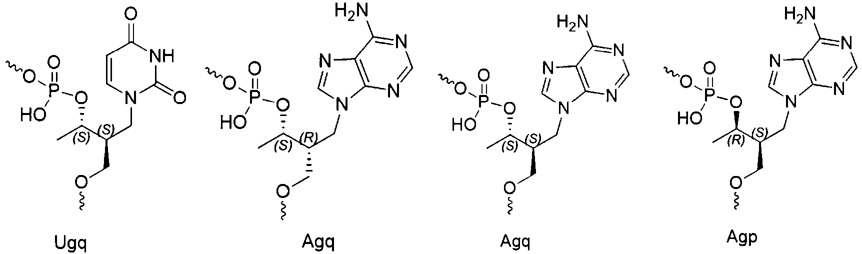

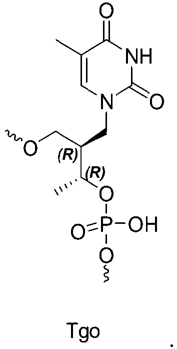

4. The single-stranded or double-stranded oligonucleotide according to claim 2, **characterized in that**: the chemical modification is selected from any one of the following structures:

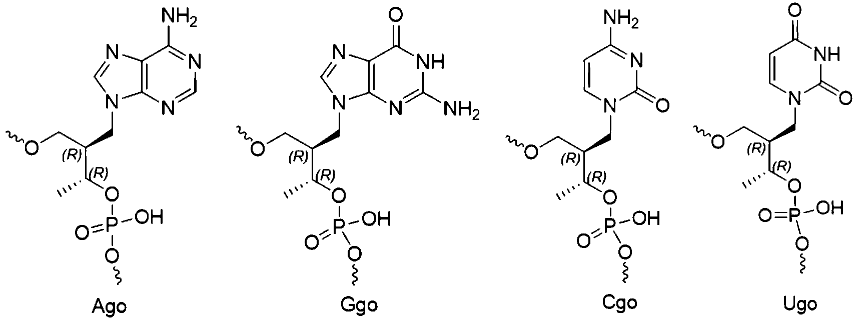

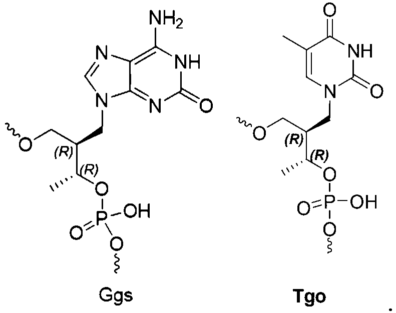

5. The single-stranded or double-stranded oligonucleotide according to any one of claims 1 to 4, **characterized in that**: the strand comprises a chemical modification represented by formula (II), (II-1), (II-2), (II-3) or (II-4) or a tautomer modification thereof at at least one nucleotide position from position 5 to position 8 of its 5' region.

6. The single-stranded or double-stranded oligonucleotide according to claim 5, **characterized in that**: the strand comprises a chemical modification represented by (II), (II-1), (II-2), (II-3) or (II-4) or a tautomer modification thereof at at least one nucleotide position of position 6 and 7 of its 5' region.

7. The single-stranded or double-stranded oligonucleotide according to claim 5, **characterized in that**: the strand is the antisense strand of a double-stranded oligonucleotide, and comprises a chemical modification represented by Ago, Ggo, Cgo, Ugo, Ggs or Tgo or a tautomer modification thereof at at least one nucleotide position of position 6 and 7 of its 5' region.

8. The single-stranded or double-stranded oligonucleotide according to claim 5, **characterized in that**: in addition to the nucleotide comprising the chemical modification represented by formula (II), (II-1), (II-2), (II-3) or (II-4) or a tautomer modification thereof, the strand further comprises at least one other modified nucleotide.

9. The single-stranded or double-stranded oligonucleotide according to claim 8, **characterized in that**: the other modified nucleotides are independently selected from: a 2'-fluoro-modified nucleotide, a 2'-alkoxy-modified nucleotide, a 2'-substituted alkoxy-modified nucleotide, a 2'-alkyl-modified nucleotide, a 2'-substituted alkyl-modified nucleotide, a 2'-deoxynucleotide, a 2'-amino-modified nucleotide, and a 2'-substituted amino-modified nucleotide.

10. The single-stranded or double-stranded oligonucleotide according to claim 8, **characterized in that**: the oligonucleotide is an siRNA, which comprises a sense strand and an antisense strand, wherein the siRNA has at least one of the following characteristics:
(i) the antisense strand comprises 2, 3, 4, 5 or 6 2'-fluoro modifications;
(ii) the antisense strand comprises 1, 2, 3 or 4 phosphorothioate internucleotide linkages;
(iii)the sense strand comprises 2, 3, 4 or 5 2'-fluoro modifications;
(iv)the sense strand comprises 1, 2, 3 or 4 phosphorothioate internucleotide linkages.

11. An siRNA conjugate, comprising the single-stranded or double-stranded oligonucleotide according to any one of claims 1 to 10 and a conjugating group conjugated to the oligonucleotide.

12. A pharmaceutical composition, comprising the single-stranded or double-stranded oligonucleotide according to any one of claims 1 to 10 or the siRNA conjugate according to claim 10, and a pharmaceutically acceptable carrier.

13. A kit, comprising the single-stranded or double-stranded oligonucleotide according to any one of claims 1 to 10, or the siRNA conjugate according to claim 11, or the pharmaceutical composition according to claim 12.

14. A compound represented by formula (IV) or a tautomer thereof, 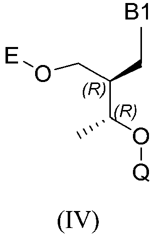
wherein, B1 is a natural nucleobase, a modified nucleobase, a universal base or an H atom;
E is a leaving group; preferably, E is MMTr or DMTr;
Q is a phosphorus-containing reactive group; preferably, Q is

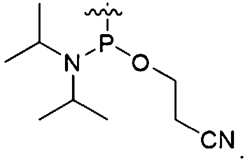

15. The compound or tautomer thereof represented by formula (IV) according to claim 14, **characterized in that**: the compound or tautomer thereof represented by formula (IV) is selected from any one of the following structures:

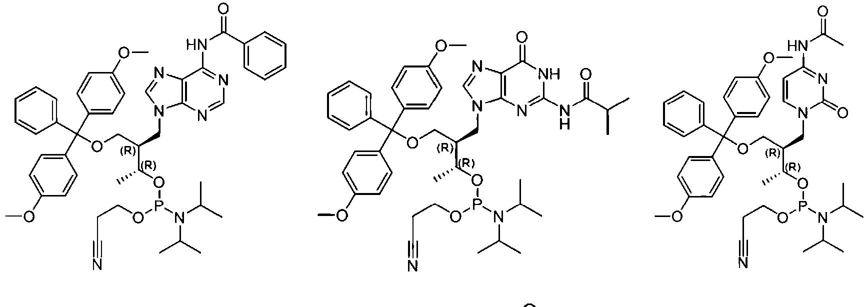

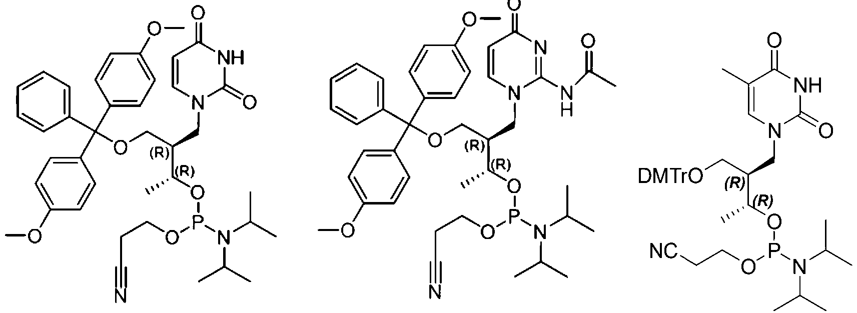

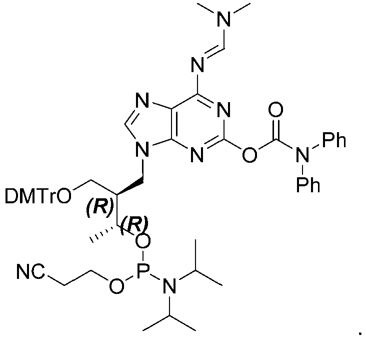

16. A method for preparing the single-stranded or double-stranded oligonucleotide according to any one of claims 1 to 10 or the siRNA conjugate according to claim 11, comprising the following steps:
1) synthesizing the compound represented by formula (IV) or a tautomer thereof according to claim 13 or 14;
2) using the compound or tautomer thereof from step 1) to synthesize the oligonucleotide or siRNA conjugate.

17. A method for inhibiting a target gene in a cell, **characterized in that**: the method comprises the step of introducing the single-stranded or double-stranded oligonucleotide according to any one of claims 1 to 10 or the siRNA conjugate according to claim 11 into the cell.

18. A method for modifying positions 2-8 of an antisense strand of an siRNA to reduce off-target activity, **characterized in that**: a chemical modification represented by general formula (II), (II-1), (II-2), (II-3) or (II-4) as defined in claim 1 or 2, or a tautomer modification thereof, is introduced into positions 2-8 of the antisense strand of an siRNA molecule.

19. The method according to claim 18, **characterized in that**: the chemical modification or tautomer modification thereof represented by general formula (II), (II-1), (II-2), (II-3) or (II-4) is selected from any one of the following structures:

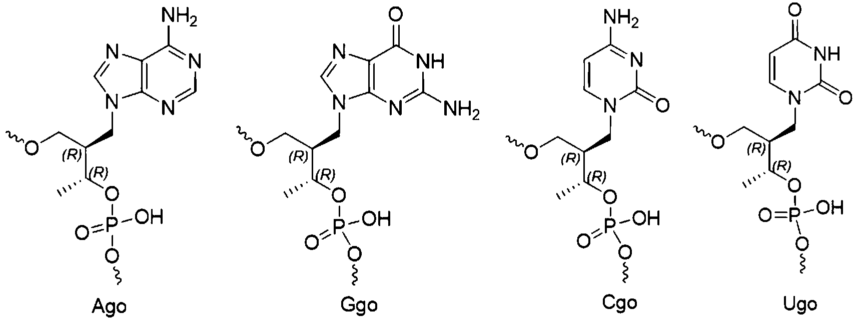

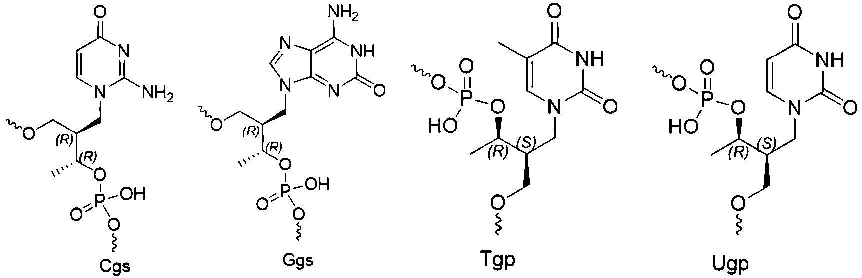

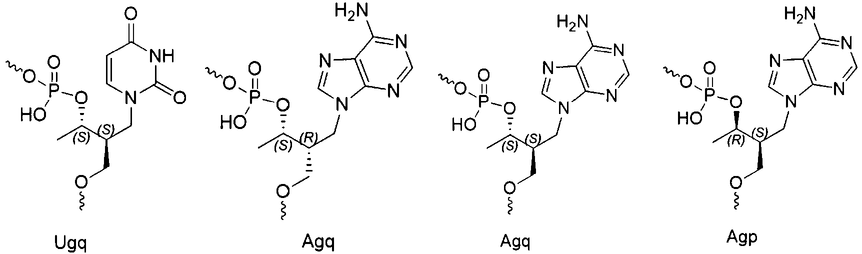

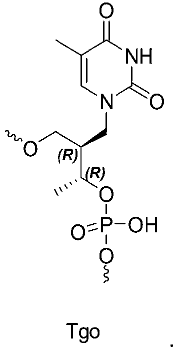

20. The method according to claim 18, **characterized in that**: the chemical modification or tautomer modification thereof represented by general formula (II), (II-1), (II-2), (II-3) or (II-4) is selected from any one of the following structures:
